# EUROPEAN PATENT APPLICATION

(11) **EP 4 417 622 A1**
(43) Date of publication of application: **21.08.2024**
(21) Application number: 22885830.4
(22) Date of filing: 24.10.2022
(51) Int. Cl.: C07K 16/28, C07K 16/46, C12N 15/13, C12N 15/63, C12N 1/15, C12N 1/19, C12N 1/21, A61K 39/395, A61P 35/00

(54) **ANTI-CD26 ANTIBODY AND USE THEREOF**

(30) Priority: 25.10.2021 CN 202111245489
(71) Applicant: Zonhon Biopharma Institute, Inc., Changzhou, Jiangsu 213125 (CN)
(72) Inventor: MA, Bruce Yong, Changzhou, Jiangsu 213125 (CN); CAO, Peipei, Changzhou, Jiangsu 213125 (CN); HANG, Jianhua, Changzhou, Jiangsu 213125 (CN); GE, Chennan, Changzhou, Jiangsu 213125 (CN); ZHANG, Tao, Changzhou, Jiangsu 213125 (CN); SUN, Fang, Changzhou, Jiangsu 213125 (CN); YAO, Xiang, Changzhou, Jiangsu 213125 (CN)
(74) Representative: Herrmann, Uwe
(86) International application number: PCT/CN2022/126880
(87) International publication number: WO 2023/071953

(57) **Abstract**

Provided are antibodies or antigen-binding fragments that bind to human CD26, bispecific T-cell engagers comprising the sequence of such antibodies or antigen-binding fragments, and applications of such antibodies or antigen-binding fragments in the preparation of drugs for the treatment of tumors with high expression of CD26.

## Description

### TECHNICAL FIELD

The present invention relates to an antibody or antigen-binding fragment that binds to human CD26 and its application in the preparation of drugs for treating tumors with high expression of CD26.

### BACKGROUND OF THE INVENTION

CD26 is a multifunctional type II transmembrane glycoprotein that can also be found in plasma in a dissolved form. CD26 usually exists as a homodimer with a monomer comprising 766 amino acids and a relative molecular mass of about 110kDa. The amino acid residues of CD26 can be divided into five parts from inside to outside: intracellular region (1-6), transmembrane region (7-28), highly glycosylated region (29-323), cysteine-rich region (324-551) and C-terminal catalytic domain (552-766). The molecular structure of CD26 is closely related to its function. CD26(DPP4) inhibitors have been used in the treatment of type 2 diabetes for decades. The expression of CD26 is significantly increased on the surface of a variety of tumor cells, such as malignant mesothelioma, renal cancer, prostate cancer, lung cancer, etc. CD26 is a valuable target for these types of tumors with high CD26 expression(CD26 is a potential biomarker and target for cancer therapy Pharmacology&Therapeutics(2019),198:135-159).

At present, the most advanced anticancer drug research targeting human CD26 is the monoclonal antibody YS110 from Y's Therapeutics, which has completed phase I/ II clinical trials. However, according to the existing literature, YS110 has been found to have low activity in preclinical studies both in vitro and in vivo. As in the literature "A humanized anti-CD26 monoclonal antibody inhibits cell growth of malignant mesothelioma via retarded G2/M cells In cycle transition, Cancer Cell Int(2016)16:35 ", after treated with 250ug/ml YS110 for 48h, the growth inhibition rate of tumor cell line was 18.3%, and the IC₅₀ value was much higher than 250ug/ml. These results indicated that its activity in vitro was low. In the patent "anti-CD26 antibody and use thereof' (Application No. CN200680034937.4), after the treatment of YS 110 in mice bearing a variety of CD26 high expression tumor cell lines, YS110 can only reduce tumor volume and alleviate tumor growth to a certain extent, but can not completely inhibit tumor growth to achieve tumor cure, showing its activity in animal models is still not ideal. Results from the completed phase 2 clinical study of YS110 have shown that YS110 is well tolerated, but disease control rates have not been as high as expected, which corresponds to the low activity of YS110 in preclinical studies.

Although the widespread distribution of CD26 has been reported in the literature, the use of YS110 is still safe, indicating that its safety in clinical use is well guaranteed. In the 31 patients of YS110 clinical trial, there was no patient death. The main side effects were infusion adverse reactions (16.1%), hiccups (9.7%) and diarrhea (6.5%). (Phase 2 Study of YS 110,a Recombinant Humanized Anti-CD26 Monoclonal Antibody,in Japanese Patients With Advanced Malignant Pleural Mesothelioma,JTO Clinincal and Research Reports(2021),2(6))

In conclusion, although the safety of CD26 has been verified in clinical trials, the existing antibodies targeting CD26 are difficult to achieve good therapeutic effects on tumor treatment.

### SUMMARY OF THE INVENTION

To solve the above problems, the first aim of the present invention is to provide a more effective and safer CD26 antibody or antigen-binding fragment.

An antibody or antigen-binding fragment specifically binding to human CD26 of the present invention comprises HCDR1 shown as SEQ ID NO:1, HCDR2 shown as SEQ ID NO:2, and HCDR3 shown as SEQ ID NO:3; as well as LCDR1 shown as SEQ ID NO:4, LCDR2 consisting of Arg Met Ser, and LCDR3 shown as SEQ ID NO:5.

The antibody or antigen-binding fragment comprises a variable region of heavy chain that is at least 90%, 91%, 92%, 93%, 94%, 95%, 96%, 97%, 98%, or 99% identical to the amino acid sequence of SEQ ID NO:6 or SEQ ID NO:8,and a variable region of light chain that is at least 90%, 91%, 92%, 93%, 94%, 95%, 96%, 97%, 98%, or 99% identical to the amino acid sequence of SEQ ID NO:7 or SEQ ID NO:9.

The antibody or antigen-binding fragment, which is one, two, three, four, five, six, seven, eight, nine, or ten amino acids of the sequence shown as SEQ ID NO:6, SEQ ID NO:7, SEQ ID NO:8, or SEQ ID NO:9 are inserted, deleted, or substituted. The amino acid substitutions are conservative amino acid substitutions.

The antibody or antigen-binding fragment comprises a variable region of heavy chain shown as SEQ ID NO:6 and a variable region of light chain shown as SEQ ID NO:7; Or a variable region of heavy chain shown as SEQ ID NO:8 and a variable region of light chain shown as SEQ ID NO:9.

The second purpose of the present invention is to provide a bispecific T cell engager (BiTE) comprising the sequence of an antibody or antigen-binding fragment targeting CD26.

The bispecific T cell engager (BiTE) comprising the sequence of an antibody or antigen-binding fragment targeting CD26 of the present invention, is formed by connecting two antigen-specific single-chain variable fragments (scFv) through a linker. One scFv targeting CD26, is formed by connecting a variable region of heavy chain and a variable region of light chain targeting CD26 through a linker; Another scFv targets CD3, is formed by connecting a variable region of heavy chain and a variable region of light chain targeting CD3 through a linker ; The scFv targeting CD26 comprises HCDR1 shown as SEQ ID NO: 1, HCDR2 shown as SEQ ID NO:2, HCDR3 shown as SEQ ID NO:3; As well as LCDR1 shown as SEQ ID NO:4, LCDR2 consisting of Arg Met Ser, and LCDR3 shown as SEQ ID NO:5.

The scFv targeting CD26 comprises a variable region of heavy chain that is at least 90%, 91%, 92%, 93%, 94%, 95%, 96%, 97%, 98%, or 99% identical to the amino acid sequence of SEQ ID NO:6 or SEQ ID NO:8,and a variable region of light chain that is at least 90%, 91%, 92%, 93%, 94%, 95%, 96%, 97%, 98%, or 99% identical to the amino acid sequence of SEQ ID NO:7 or SEQ ID NO:9.

The scFv targeting CD26 comprises a sequence wherein one, two, three, four, five, six, seven, eight, nine, or ten amino acids of the sequence shown as SEQ ID NO:6, SEQ ID NO:7, SEQ ID NO:8, or SEQ ID NO:9 are inserted, deleted, or substituted. The amino acid substitutions are conservativ amino acid substitutions.

The scFv targeting CD26 comprises a variable region of heavy chain shown as SEQ ID NO:6 and a variable region of light chain shown as SEQ ID NO:7; Or comprises a variable region of heavy chain shown as SEQ ID NO:8 and a variable region of light chain shown as SEQ ID NO:9.

The scFv targeting CD3 is derived from OKT-3, L2K, TR66, UCHT1, SP34, IORT3, Catumaxomab, Blinatumomab, Solitomab and other CD3 antibody sequences known in the art. Such as the anti-CD3 variable region of heavy chain shown as SEQ ID NO: 10 and the anti-CD3 variable region of light chain shown as SEQ ID NO: 11.

The linker connecting the variable region of heavy chain and the variable region of light chain in scFv is selected from the linkers commonly used in the art. For example, KESGSVSSEQLAQFRSLD, EGKSSGSGSESKST, GSTSGGGSGGGGSS, GSTSGSGKPGSGEGSTKG, (GGGGS)n, where n is an integer from 1 to 5, preferably n is an integer from 1 to 3, preferably n is 3, shown as SEQ ID NO: 12.

The linker connecting two scFvs is selected from linkers commonly used in the art, such as (GGGGS)n, where n is an integer from 1 to 5, preferably an integer from 1 to 3, preferably n is 1, shown as SEQ ID NO: 13.

As preferred, the bispecific T cell engager comprising the sequence of an antibody or antigen-binding fragment targeting CD26 is shown as SEQ ID NO: 14(amino acid sequence of 18G272 BiTE) or SEQ ID NO:15(amino acid sequence of 19G294 BiTE).

The invention also relates to a nucleotide sequence encoding an amino acid sequence of the antibody or antigen-binding fragment. The invention also relates to a vector comprising the nucleic acid, a host cell comprising the nucleic acid.

The present invention also relates to methods for producing antibodies or antigen-binding fragments , comprising culture of the host cell, recovery of the antibody or antigen-binding fragments from the culture.

The present invention also relates to a pharmaceutical composition comprising the antibody or antigen-binding fragment, which also comprises a pharmaceutically acceptable excipients.

The present invention also relates to a method of treating tumors, comprising administering to a patient an effective amount of the antibody or antigen-binding fragment; The tumors highly expressing CD26, includes but not limited to renal cell carcinoma, mesothelioma, lung cancer, liver cancer, prostate cancer, etc.The antibody or antigen-binding fragment is administered alone or in combination with other anticancer agents.

### The application has the following advantages over the prior art:

### First, the mouse-derived antibodies screened in this application have obvious advantages.

Example 1 shows that No. 62 mouse derived antibody screened in this application has a much higher lysis intensity to human tumor cells than other mouse derived antibodies. Moreover, the killing effect of the mouse derived antibody screened in this application on human tumor cells has been equivalent to that of the humanized antibody YS 110 which has completed the clinical phase II, which fully proves the advantages of the mouse derived antibody.

In addition, the data of examples 5, 8 and 10 show that the chimeric antibody or humanized antibody based on the variable region of No. 62 mouse antibody has better dot blot activity and better affinity with antigen than the corresponding antibody derived from other mouse antibodies.

Compared with BiTE derived from other mouse derived antibodies, the BiTE based on the variable region of No. 62 mouse antibody in this application shows better effectiveness. Example 11 shows when comparing the BiTE mediated cytotoxicity of PBMC to different target cells in vitro, the BiTE derived from No. 62 mouse antibody of the application has better killing effect on a variety of tumor cells than the BiTE derived from CD26 antibody YS 110. The results of Examples 17-22 show that the proposed BiTE can significantly inhibit tumor growth in a variety of animal tumor models, and can completely inhibit tumor growth in some models, which is significantly better than the BiTE derived from YS 110.

Furthermore, the BiTE based on the variable region of No. 62 mouse antibody of this application shows better safety (Example 13) and a lower risk of causing cytokine storm than BiTE derived from other mouse antibody.

### Second, the proposed bispecific T cell engager targeting CD26 is more effective and safer than available full-length monoclonal antibodies targeting CD26.

In terms of effectiveness, the data of Example 11 show that BiTE of this application has a better killing effect on a variety of CD26 positive tumor cells than the full-length IgG antibody YS110. The data from Examples 23 and 24 show that the BiTE of this application has significantly better tumor growth inhibition than the full-length IgG antibody YS 110 in a variety of animal tumor models.

In terms of safety, the data of Example 12 show that anti-CD26 full-length IgG antibody does not effectively induce T cell activation. The BiTE of this application does not cause significant T cell activation before reaching the tumor site. After binding to the tumor cells, the BiTE of this application can cause the activation of T cells around the tumor tissue, and then kill the tumor cells, and improve the safety. The data from examples 23 and 24 show that the BiTE of this application shows significantly better safety than the full-length IgG antibody YS110 in a variety of animal tumor models.

### Third, the bispecific T cell engager targeting CD26 in the present application shows superior efficacy and safety compared to existing first-line treatment options for related tumors, such as BAVENCIO for renal cancer (Examples 15, 22, 23).

### BRIEF DESCRIPTION OF THE DRAWINGS

Figure 1: dot blot assay of humanized bispecific antibodies
Figure 1a shows the dot-blot assay of humanized bispecific antibody 18G272 and 18G278, Figure 1b shows the dot-blot assay of humanized bispecific antibody 19G294
Figure 2: antibody specific binding to target cell antigen measured by flow cytometry
Figure 3: 19G294 binding to both CD26 and CD3 proteins
Figure 4: Cytotoxic effect of PBMC against PBMC mediated by a humanized bispecific antibody
Figure 5: Tumor volume in NOD/SCID mice subcutaneously bearing PC3 cells with the administration of 18G272 or 19G294
Figure 6: Body weight in NOD/SCID mice bearing subcutaneous PC-3 cells with the administration of 18G272 or 19G294
Figure 7: Tumor volume in NOD/SCID mice bearing subcutaneous NCI-H596 cells with the administration of 18G272
Figure 8: Tumor volume in NOD/SCID mice bearing subcutaneous NCI-H226 cells with the administration of 19G294
Figure 9: Body weight in NOD/SCID mice bearing subcutaneous NCI-H226 cells with the administration of 19G294
Figure 10: Tumor volume in NOD/SCID mice bearing subcutaneous A498 cells with the administration of 19G294
Figure 11: Tumor volume in NOD/SCID mice bearing subcutaneous OS-RC-2 cells with the administration of 17G29 or 19G295
Figure 12: Tumor volume in NOD/SCID mice bearing subcutaneous OS-RC-2 cells with the administration of18G272 or 19G295
Figure 13: Animal survival in NOD/SCID mice bearing subcutaneous OS-RC-2 cells with the administration of 18G272 or 19G295
Figure 14:Tumor volume in NOD/SCID mice bearing subcutaneous OS-RC-2 cells with the administration of 18G272
Figure 15: Tumor volume in NOD/SCID mice bearing subcutaneous OS-RC-2 cells with the administration of 19G294
Figure 16: Tumor volume in NOD/SCID mice bearing subcutaneous OS-RC-2 cells with the administration of 19G294 or 21G587 in different doses
Figure 17: Body weight in NOD/SCID mice bearing subcutaneous OS-RC-2 cells with the administration of 19G294 or 21G587 in different doses
Figure18: Tumor volume in NOD/SCID mice bearing subcutaneous OS-RC-2 cells with the administration of 19G294 or BAVENCIO in combination with Axitinib
Figure 19: Tumor volume in NOD/SCID mice bearing subcutaneous OS-RC-2 cells with the administration of 19G294 or BAVENCIO in combination with Axitinib on Day 26 after cell inoculation
Figure 20: Tumor volume in NOD/SCID mice bearing subcutaneous A498 cells with the administration of 19G294 or BAVENCIO in combination with Axitinibon Day 26 after cell inoculation
Figure 21: Tumor weight in NOD/SCID mice bearing subcutaneous A498 cells with the administration of 19G294 or BAVENCIO in combination with Axitinib on Day 65 after cell inoculation

### DETAILED DESCRIPTION OF THE INVENTION

Unless clearly defined herein, technical terms used herein have meanings generally understood by one of ordinary skill in the art.

Singular words such as "a, an" and "the " include their corresponding plural form.

The term "or" means "and/or" and can be used interchangeably with "and/or".

The term "CD26" is also known as DPP4 (dipeptidyl peptidase 4). The amino acid sequence of human CD26 can be found in Genbank with accession number NP_001926.2. Its cDNA sequence can be found in Genbank with the accession number NM_001935.3.

The term "conservative amino acid substitution" means the replacement of an original amino acid with a new amino acid that substantially does not change the chemical, physical, and/or functional properties of the antibody or fragment, such as its binding affinity to CD26. Common conserved substitutions of amino acids are well known in the art, for example, Ala can be conservatively substituted with Gly or Ser; Arg could be conservatively replaced by Lys or His. Asn can be conservatively replaced by Gln, His, etc. The term "affinity" refers to the strength of the interaction between an antibody and an antigen. The variable region of antibody interacts with antigen by non-covalent force. The more interactions, the stronger the affinity.

The term "antibody" refers to a family of immunoglobulins that can specifically bind the corresponding antigen noncovalently, reversibly. For example, naturally IgG antibodies are tetamers that comprises at least two heavy chains and two light chains linked to each other by disulfide bonds. Each heavy chain consists of a variable region of heavy chain (VH) and a constant region of heavy chain (CH). The constant region of heavy chain consists of three domains CH1, CH2, and CH3. Each light chain consists of a variable region of light chain (VL) and a constant region of light chain (CL). The constant region of light chain consists of one domain, CL. VH and VL can be further subdivided into complementarity determining regions (CDR, also known as hypervariable regions) with high variability, and a more conserved framework region (FR). Each VH and VL consists of three CDRs and four FRs, arranged in the following order from amino-terminal to carboxyl terminal: FR1, CDR1, FR2, CDR2, FR3, CDR3 and FR4. HCDR1, HCDR2 and HCDR3 are three complementarity determining regions of heavy chain, and LCDR1, LCDR2 and LCDR3 are three complementarity determining regions of light chain. The location of the CDR and framework region may be determined using various numbering systems well known in the art, such as Kabat, Chothia, IMGT, etc., IMGT is used in this application. The variable region of heavy chain (VH) and variable region of light chain (VL) regions are responsible for antigen recognition, especially complementarity-determining regions (CDR), which are usually specific for different epitopes of antigens. The constant region is primarily responsible for effector functions.

The term "antigen-binding fragment" refers to an antigen-binding fragment of an antibody, that is, an antibody fragment that retains the ability to bind specifically to an antigen, such as a fragment that retains one or more CDR regions, including but not limited to Fab fragments, FV fragments, double antibodies, linear antibodies, single chain variable fragment, nanoantibodies, multi-specific antibodies, etc.

The term "monoclonal antibody" refers to a population of antibodies that is essentially homogeneous, in the sense that the amino acid sequences of the antibody molecules contained in the population are identical, except for a small number of present mutations that may occur naturally. In contrast, polyclonal antibodies typically include many different antibodies with different amino acid sequences in their variable domains, particularly their complementarity determining regions. Monoclonal antibodies may be obtained by methods known to one skilled in the art. This application uses hybridoma technique to obtain monoclonal antibodies, which is one of the many methods known to obtain monoclonal antibodies.

The term "murine antibody" refers to an antibody consisting only of a rat or mouse immunoglobulin sequence.

The variable region of "chimeric antibody" is derived from non-human (e.g., murine) antibody sequences, and the remainder is derived from human antibody sequences.

The CDR region of "humanized antibody" is derived from non-human (e.g., murine) antibody sequences, and the remainder is derived from human antibody sequences.

The term "bispecific antibody" is an antibody that has two specific antigen-binding sites of the same or different antigens.

The term "BiTE" is a bispecific antibody, full name "bispecific T cell engager", formed by connecting two single chain variable fragments with antigen specificity through a linker. One scFv targets tumor-associated antigens and the other scFv targets CD3 on T cells. The single chain variable fragment (scFv) is formed by connecting the variable region of heavy chain and the variable region of light chain through a linker. Linker is an amino acid sequence used to link different protein fragments. There have been many studies on the design and selection of linker. In BiTE, the linker connects a variable region of heavy chain to a variable region of light chain to form scFv, and also concatenates two scFvs and allows them to rotate freely. It is generally composed of GGGGS repeats (S for serine, G for glycine). Glycine with small molecular weight and short side chain can increase the flexibility of side chain. The stronger hydrophilicity of serine may increase the hydrophilicity of the peptide chain. Optimizing the number of repeats not only helps the light and heavy chains in the scFv to form correct conformation, but also affects the distance between the two scFvs to promote the optimal interaction between T cells and target cells at the immune synapse (Zhouchong, etc. Research progress of bispecific single-chain antibody BiTE [J]. Chinese Journal of Biology,2018 "). KESGSVSSEQLAQFRSLD, EGKSSGSGSESKST, GSTSGGGSGGGGGGSS (US20180326032), GSTSGSGKPGSGEGSTKG(Preclinical Development of Bivalent Chimeric Antigen Receptors Targeting Both CD 19 and CD22) are commonly used to connect the variable region of heavy chain with the variable region of light chain in scFv. In a specific embodiment of the present application, the flexible linker connecting a heavy chain and a light chain to form scFv is composed of three GGGGS, and the flexible linker connecting two scFv is GGGGS. The linker can be selected and determined by one skilled in the art according to the teaching of the prior art through conventional experimental methods and finite tests from known linkers. The number of repeats of GGGGS in the two flexible linkers can be optimized, or other flexible linkers other than GGGGS can be used.

CD3 is a component of T-cell signaling. When BiTE binds to both T cells and tumor cells, T cells are activated, which promotes CD8+T cells to directly secrete perforin and granzyme, and promotes CD4+T cells to secrete cytokines to recruit and activate killer T cells, thereby killing tumor cells. The CD3 antibody sequence can be selected from OKT-3, L2K, TR66, UCHT1, SP34, IORT3, Catumaxomab, Blinatumomab, Solitomab, WBP3311_2.306.4(Patent CN201880061333.1) and other known CD3 antibody sequence in the art. In a specific embodiment of the application, the CD3 antibody sequence of Solitomab, WBP3311_2.306.4 is used, and the person skilled in the art can select and determine from other known CD3 antibodies by conventional experimental methods and a limited number of tests, rather than being limited to a specific CD3 antibody sequence of the application.

The term "chimeric BiTE bispecific antibody" refers to murine CD26 scFv connected to humanized CD3 scFv by an linker.

The terms "administration", "administering", "treating", and "treatment" refer to the contact of an exogenous agent, therapeutic agent, or diagnostic agent with the tissues, organs, cells, or biological fluids of a subject. "Treatment" refers to slowing or preventing the development of clinical symptoms of the disease, or to alleviating or improving at least one physical parameter, or to preventing the progression of the disease.

### Example 1. Preparation and selection of hybridoma cell lines against human CD26

### Step 1: Immunize animals

According to the CD26 cDNA sequence published in GenBank (GenBank accession number: NM_001935.3), the rCD26 expression vector was designed, the histidine tag was introduced at the 5 'end of the codon, the whole gene was synthesized, connected to the pCHO1.0 plasmid, and expressed in CHO-S cells. The target CD26 protein was purified. The prepared recombinant CD26 protein was used to immunize female BALB/c mice according to the general immunization schedule. For specific immunization, please refer to the << Making and Using Antibodies: A Practical Hankbook>>. Indirect ELISA was used to detect the serum titer of immunized mice, and the immunized mouse with the highest serum titer were selected for fusion experiments with spleen cells and myeloma cells.

### Step 2: Cell fusion

### (1). Preparation of splenic cells

Eyeballs of immunized mouse were removed and blood was collected. The mouse was sacrificed by cervical vertebra amputation and immersed in 75% (v/v) alcohol for 10 minutes. The spleen was removed in a sterile operating table, placed in a cell screen, fully ground the cells, passed through the screen, centrifuged and washed with sterile 1640 medium (purchased from Gibco Company) for several times, and the cells were resuspended to make single cell suspension. And count and set aside.

### (2). Preparation of feeder cells

A female BALB/c mouse aged 8-10 weeks was selected, and negative serum was obtained by enucleate the eyeball. The mouse was sacrificed by cervical vertebra amputation and immersed in 75% (v/v) alcohol for 10 minutes. The abdominal skin was aseptically uncovered to expose the peritoneum, and approximately 10mL of 1640HT medium (purchased from SIGMA) was injected into the abdominal cavity of mouse using a syringe, the abdomen was gently massaged and blown several times. The medium containing macrophages was aspirated and injected into 20% 1640HAT medium for later use.

A female BALB/c mouse aged 2 to 3 weeks was sacrificed by cervical vertebra amputation and immersed in 75% (v/v) alcohol for 10 minutes. Thymocytes were aseptically removed, placed in a cell screen, ground, passed through the screen, and thymocytes were obtained and placed in 20% 1640HAT medium containing macrophages as described above for use.

### (3). Cell fusion

Mouse myeloma cell line SP2/0 in logarithmic growth phase was selected, collected and counted. About 10⁸ of the above spleen cells and 2×10⁷ of the above SP2/0 cell lines were added to the fusion tube and mixed. After centrifugation at 1000 rpm for 10 minutes, the supernatant was discarded, and the fusion tube was placed on the palm and gently rubbed back and forth to loosen the precipitate. Add 1mL of preheated PEG1450(polyethylene glycol 1450, purchased from SIGMA) slowly and then quickly within 60 seconds, terminate by adding 30mL of 1640HT medium, centrifuge at 1000rpm for 10 minutes, remove the supernatant, gently rub to loosen the precipitate, and add to the 20% 1640HAT medium obtained in step 2.

The above HAT medium was mixed thoroughly, and 200µL/ well was sub-packed into a 96-well cell culture plate and incubated in a cell incubator at 37°C with 5% CO₂. One week later, 10% 1640HT medium was used to replace 20% 1640HAT medium, and the supernatant was taken after 3 days for assay.

### Step 3: Screening of hybridoma cell lines specific for CD26

(1) Preparation of detection plate: the recombinant CD26 protein was diluted to 1µg/ml with CB coating solution, coated into 96-well ELISA plate, 100µl/well, coated at 2-8 °C overnight, washed once and dry; The cells were blocked with 2% BSA in PBST buffer (200µl/ well) and then blocked at 37°C for 2 hours. Dry and set aside.
(2). Screening of positive clones: The cell culture supernatant was added to the test plate, 100µl/well, incubated at 37°C for 30 minutes, washed and dried. Then 100µl/well diluted HRP-labeled sheep anti-mouse IgG was added, incubated at 37°C for 30 minutes, washed and dried, and 100µl/ well TMB chromogenic solution was added, incubated at 37°C in the dark for 15 minutes. The reaction was terminated by adding 50µl of 2M H₂SO₄ to each well, and the values were read at OD₄₅₀. The positive wells was determined as follows: OD₄₅₀ value/negative control value ≥2.1. Positive clones were selected for cell cloning screening. After three to four rounds of cloning and screening, the positive rate of monoclonal cell line was 100%, which was defined as stable cell line.

### Step 4: Evaluation of mouse monoclonal antibody against CD26

In the 786-0 cell reaction system, 786-0 cells were labeled with green fluorescent signal by fluorescent dye Calcein-AM. The 786-0 cells were seeded into U-type 96-well cell culture plate at a cell concentration of 6×10⁵cells/ml, 50µl per well, and each group was set up with 3 parallel holes. Add 50ul CD26 antibody at a final concentration of 100ng/ml to the sample wells, add 50ul medium control to the blank control wells, add 50ul Triton X-100 at a final concentration of 1% to the positive control wells, and incubate at 37 °C for 30min. PBMC cells (peripheral blood mononuclear cells) at a concentration of 6×10⁶cells/ml were added at E/T ratio of 10:1. The reaction system was incubated at 37°C in carbon dioxide for 5h. After the reaction, centrifuged and placed the supernatant in a new 96-well plate. After centrifugation again, 80µl of the supernatant was placed in a black 96-well plate. The detection was performed with a microplate reader at 470nm excitation wavelength and 515nm emission wavelength. The formula of cell lysis rate = (Vₛₐₘₚₗₑ-V_{vehicle control})/(V_{TritonX-100}-V_{vehicle control})× 100%. (Vₛₐₘₚₗₑ is the mean value of the fluorescence signal reading measured at excitation and emission wavelengths in the drug treatment group, V_{vehicle control} is the mean value of the fluorescence signal reading measured at excitation and emission wavelengths in the blank control group,V_{TritonX-100} is the mean value of fluorescence signal reading measured at excitation wavelength and emission wavelength in positive control group.)

**Table 1 Antibody mediated PBMC cytotoxic effect on 786-0 cells**

| | | | | | | |
|---|---|---|---|---|---|---|
| antibody number | 72 | 160 | 62 | 51 | 212 | YS110 |
| cell lysis rate | 8.6% | 13.0% | 53.1% | 12.3% | 11.3% | 56.6% |

The results showed that at the concentration of 100ng/ml, No. 62 mouse antibody mediated lysis rate of 786-0 by PBMC was 53.1% after 5h , which was much higher than that of other murine monoclonal antibodies , such as NO.72, 160, 51, 212 mouse antibody , and was close to the humanized antibody YS110.

786-0 is a human tumor cell. Theoretically, the successful humanized antibody has a much stronger affinity with human cells than the parental murine antibody, and will also have a stronger lysis of the tumor cell. The killing effect of No. 62 mouse antibody in this application on human tumor cells has been equivalent to that of the humanized antibody YS110 which has completed the clinical phase II, proving the advantages of No. 62 mouse antibody. It is reasonable for one skilled in the art to expect that the humanized antibody derived from No. 62 mouse antibody will be superior to No. 62 mouse antibody itself and to YS110 in killing human tumor cells, which has also been verified in Example 11.

### Example 2. Screening of hybridoma cell lines by sequencing of variable region sequences

### Step 1. Total RNA extraction from CD26 hybridoma cells

The hybridoma cell lines were passaged into T75 culture flasks and cultured until the cells fusion reached about 90%. Cells were collected. Total RNA was extracted from the monoclonal hybridoma cell lines using an RNA extraction kit (purchased from Roche). Then, the total RNA was used as a template to amplify the first strand of cDNA by cDNA reverse transcription kit (purchased from Thermo), and the reaction products were stored at -20 °C , or -70 °C for long-term storage.

### Step 2.PCR amplification of variable region of heavy and light chain genes

The first strand cDNA of hybridoma cells was used as template, and 1µl of cDNA, 5µl of 10×PCR buffer, 1µl of upstream and 1µl of downstream primers (25pmol), dNTP1µl, 1µl of 25mmol/L MgCl₂, and 39µl of H₂O were added respectively to form the 50µl reaction system. After initial denaturation at 95 ° C for 10min, 1µl of Taq enzyme was added and entered into a temperature cycle for PCR amplification. The reaction condition was 94 ° C. Denaturation was performed at 58 ° C for 1min. There were 30 cycles of annealing for 1min and extension at 72 ° C for 1.5min, followed by kept at 72°C for 10min. 5µl of the PCR product was subjected to 1.2% agarose gel electrophoresis.

### Step 3. Cloning and sequencing of variable region genes of heavy and light chain

According to the instruction of pGM-T Fast ligation kit (Beijing Tiagen Biochemical Technology Co., Limited, VT207-02), the variable region genes of heavy and light chain were ligated with pGM-T vector, respectively, transformed into Escherichia coli Top10 competent cells, screened by Blue-White Screening and cultured at 37°C for 12-16 hours.

The resulting white colonies were inoculated in 1-5 mL LB medium containing ampicillin at a final concentration of 100µl and shaken at 37 ° C for 3-4h. PCR was used to screen for clones with correct sequence insertion.The positive clones were screened by PCR and sequenced. The amino acid sequences of variable region of heavy chain and light chain and CDR region of the antibody were obtained by IMGT comparative analysis of the computer network gene library.

NO. 62 mouse antibodies comprised HCDR1 shown as SEQ ID NO:1, HCDR2 shown as SEQ ID NO:2, and HCDR3 shown as SEQ ID No :3, and LCDR1 shown as SEQ ID NO:4, LCDR2 composed of Arg Met Ser, where Arg represents arginine, Met represents methionine, and Ser represents serine, and LCDR3 shown as SEQ ID NO:5. Its variable region of heavy chain is shown as SEQ ID NO:31, and its variable region of light chain is shown as SEQ ID NO:32.

### Example 3. Design of chimeric BiTE bispecific antibody

Bispecific antibodies targeting both CD26 and CD3 were designed based on the heavy and light chain sequences of NO.72, 160, 62, 51 murine monoclonal antibodies obtained from Example 1 and sequenced by the methods described in Example 2:
The variable region of light chain targeting CD26 and variable region of heavy chain targeting CD26 were connected to form the CD26 scFv regions by a short peptide(e.g. SEQ ID NO: 12) . The sequences of scFv regions derived from NO.72, 160, 62, 51 mouse antibody were shown as SEQ ID NO:16, SEQ ID NO:17, SEQ ID NO:18 , SEQ ID NO:19, respectively.

The variable region of heavy chain targeting CD3 ( e.g., SEQ ID NO:10 ) and variable region of light chain targeting CD3 (e.g., SEQ ID NO:11) were connected to form the CD3 scFv region by a short peptide(e.g. SEQ ID NO:12) . Four CD26 scFv regions are respectively fused to the CD3 scFv regions with a short peptide (e.g. SEQ ID NO:13) to form four BiTEs. The four BiTEs derived from NO.72, 160, 62, 51 mouse antibody were 17G105 , 17G108 , 17G61 , 17G131 , shown as SEQ ID NO:20, SEQ ID NO:21, SEQ ID NO:22, SEQ ID NO:23, respectively.

### Example 4. Expression plasmid construction, expression and purification of anti-CD26-CD3 chimeric bispecific antibody

The mouse (Musmusculus)IgG kappa signal peptide (e.g., SEQ ID NO:24) was added upstream of the CD26 scFv amino acid sequence (SEQ ID NO:16-19) . The optimized CD26 scFv gene sequence (SEQ ID NO: 25-28) was obtained according to the codon preference of mammalian cells CHO. And the AvrII restriction site and kozak sequence were introduced upstream. The scFv gene sequence of Solitomab anti CD3 was also optimized (SEQ ID NO:29). The linker gene(such as SEQ ID NO:30) was added upstream of the anti CD3 scFv gene, and the stop codon and BstZ17I restriction site were added downstream of the anti CD3 scFv gene. The AvrII restriction site, kozak sequence, optimized signal peptide, anti CD26 scFv, linker, anti CD3 scFv, stop codon and BstZ17I restriction site sequence were fused together to form four kinds of chimeric BiTE genes, which were synthesized and constructed into pUC57 plasmid. Several long-term preservation plasmids were formed, named pUC57-17G105, pUC57-17G108, pUC57-17G61 and pUC57-17G 131, respectively.

The backbone vector pCHO 1.0 was purchased from Thermo Fisher. "Expression box 1" was removed by SfiI digestion and self-cycled with T4 ligase to form the pZHK2.0 vector. The target BiTE genes were amplified and the PCR products were purified after 1% agarose electrophoresis. The purified PCR products and the pZHK 2.0 vector were digested by AvrII and BstZ17I.

T4 ligase ligated the double-digested PCR product into the pZHK 2.0 vector and transformed into Top10 competent cells, which were plated on LB plates containing kanamycin resistance and cultured overnight at 37°C. The next day, positive clones were screened and sequenced, which were completely consistent with the expected sequence.

Mammalian CHO cell line stably and highly expressing the CD26-CD3 chimeric BiTE antibody was seeded in Dynamis medium (A2617501, Thermo Fisher) , and fed batch culture was performed at 37 °C, 8% CO₂, and 130rpm. The supernatant of the culture medium was centrifuged at 12000rpm for 15min at low-temperature. And then the supernatant was filtered through a 0.45µm filter membrane, followed by chromatography purification. The size of the target product obtained was about 55kDa, which was the correct size.

### Example 5. The affinity detection of anti-CD26-CD3 chimeric bispecific antibody with CD26 protein and CD3 protein

The affinity of the chimeric antibody to the antigen protein CD26 was detected by Fortebio Intermolecular Interaction Octet QK^{e} instrument. First, the amino-coupled biosensor AR2G was activated with EDC/s-NHS, and the activated biosensor AR2G was solidified in CD26 protein solution diluted with 10mM sodium acetate (pH 5.0) solution. The biosensor solidified with CD26 was quenched in 1M ethanolamine (pH8.5) solution. Then equilibrated in 1× kinetic solution. After equilibration, it was bound to a humanized bispecific antibody solution. It was dissociated in 1× kinetic solution. Fortebio Data Analysis 8.0 software was used to analyze the data and calculate the affinity constant (KD), association rate constant (kon) and dissociation rate constant (kdis). The Response value represents the number of antibody molecules that can bind to the antigen. Kon indicates the binding speed of the antibody to the corresponding receptor. Kdis represents the dissociation speed of antibody from corresponding receptor. NA indicates no valid data.

**Table 2 Determination of the affinity of chimeric antibodies to CD26 protein**

| Sample No | Detection Concentration (nM) | Response | KD (M) | kon(1/Ms) | kdis(1/s) |
|---|---|---|---|---|---|
| 17G61 | 181.8 | 0.8493 | 1.73E-08 | 1.91E+04 | 3.31E-04 |
| 17G105 | 181.8 | 0.4979 | 3.93E-08 | 3.20E+04 | 1.26E-03 |
| 17G131 | 181.8 | 0.1496 | 4.36E-08 | 1.90E+04 | 8.29E-04 |
| 17G108 | 181.8 | 0.0395 | NA | NA | NA |

According to the above data, the chimeric antibody can bind CD26 and dissociate from CD26. 17G61(anti-CD26-CD3 chimeric bispecific antibody derived from No. 62 mouse antibody) had the highest response value with CD26 protein, indicating that more 17G61 molecules binded to CD26 protein at the same concentration. When 17G105(anti-CD26-CD3 chimeric bispecific antibody derived from No. 72 mouse antibody) and 17G131(anti-CD26-CD3 chimeric bispecific antibody derived from No. 51 mouse antibody) binded to CD26 protein, the response values were significantly lower than those of 17G61. The KD value of 17G61 with CD26 is smaller, so 17G61 has a stronger affinity with CD26 protein.

The affinity of chimeric antibodies to antigen protein CD3 was determined by Fortebio Intermolecular Interaction Octet QK^{e} instrument. First, the amino-coupled biosensor AR2G was activated with EDC/s-NHS, and the activated biosensor AR2G was solidified in CD3 protein solution diluted with 10mM sodium acetate (pH 5.0) solution. The biosensor solidified with CD3 was quenched in 1M ethanolamine (pH8.5) solution. Then equilibrated in 1× kinetic solution. After equilibration, it was bound to bispecific antibody solution. They were dissociated in 1× kinetic solution. Fortebio Data Analysis 8.0 software was used to analyze the data and calculate the affinity constant value (KD).

**Table 3 Determination of the affinity of chimeric antibodies to CD3 protein**

| Sample No | Detection Concentration (nM) | Response | KD (M) | kon(1/Ms) | kdis(1/s) |
|---|---|---|---|---|---|
| 17G61 | 181.8 | 0.2591 | 8.87E-09 | 1.79E+05 | 1.59E-03 |
| 17G105 | 181.8 | 0.1357 | 2.18E-09 | 3.09E+05 | 6.72E-04 |

According to the above data, 17G61 had the highest response value with CD3, which represented that more 17G61 molecules combined with CD3 protein at the same concentration. Binding of 17G105 to CD3 protein resulted in a lower response value than 17G61. These results indicate that 17G61 can bind CD3 and dissociate from CD3 protein.

### Example 6. Humanization of Anti-CD26 mouse antibodies

NO. 62 and 212 murine antibody in Example 1, which have high CD26 affinity and strong killing activity against CD26 high expressing tumor cells, were humanized. Humanization analysis was performed on the variable regions of heavy chain (shown as SEQ ID NO: 31) and the variable regions of light chain (shown as SEQ ID NO: 32) of NO. 62 mouse antibody, as well as the variable regions of heavy chain (shown as SEQ ID NO: 33) and the variable regions of light chain (shown as SEQ ID NO: 34) of NO. 212 mouse antibody. After computer calculating and predicting, a number of humanized heavy and light chain combinations were obtained from the above two antibodies. Four different heavy and light chain amino acid sequences were obtained after humanization of NO. 62 mouse antibody: 18G272-VL(SEQ ID NO: 7), 18G272-VH(SEQ ID NO: 6), 19G292-VL(SEQ ID No: 35), 19G292-VH(SEQ ID NO: 36), 19G293-VL(SEQ ID NO: 37), 19G293-VH(SEQ ID NO: 38),19G294-VL(SEQ ID NO: 9), 19G294-VH(SEQ ID NO: 8).

Four different heavy and light chain amino acid sequences were obtained after humanization of NO. 212 mouse antibody: 18G278-VL(SEQ ID NO: 39), 18G278-VH(SEQ ID NO: 40), 19G295-VL(SEQ ID NO: 41), 19G295-VH(SEQ ID NO: 42), 19G296-VL(SEQ ID NO: 43), 19G296-VH(SEQ ID NO: 44), 19G297-VL(SEQ ID NO: 45), 19G297-VH(SEQ ID NO: 46).

The humanized YS 110 antibody was used as a positive control, and its heavy and light chain sequences are as follows: YS110-VL(SEQ ID NO: 47), YS110-VH(SEQ ID NO: 48).

### Example 7. Expression plasmid construction, stable expression and purification of a humanized bispecific antibody

The heavy and light chain of each humanized CD26 antibody (18G272, 19G292, 19G293, 19G294, 18G278, 19G295, 19G296, 19G297) were linked by a short peptide (SEQ ID NO: 12) respectively, and another short peptide(SEQ ID NO: 13) was added downstream. The optimized CD26 scFv gene sequence (SEQ ID NO: 49-56) was obtained after optimization, the AvrII restriction site and kozak sequence were introduced upstream, the CD3 scFv gene (SEQ ID NO: 29), stop codon, and BstZ17I restriction site were added downstream. The humanized CD26-CD3 BiTE gene sequence was obtained, synthesized and constructed into pUC57 plasmid. They were named pUC57-18G272, pUC57-19G292, pUC57-19G293, pUC57-19G294, pUC57-18G278, pUC57-19G295, pUC57-19G296, pUC57-19G297.

At the same time, the heavy and light chain of YS 110 antibody were also linked by a short peptide (SEQ ID NO: 12), another short peptide (SEQ ID NO: 13) was added downstream, then the AvrII restriction site and kozak sequence were introduced upstream. The CD3 scFv gene (SEQ ID NO: 29), stop codon, and BstZ17I restriction site were added downstream. The humanized CD26-CD3 BiTE gene sequence was obtained, synthesized and constructed into pUC57 plasmid, named pUC-17G29.

In addition, The heavy and light chain of CD3 antibody from WBP3311_2.306.4 were linked by short peptide (SEQ ID NO: 12) to form a scFv (SEQ ID NO: 57) .The heavy and light chain of 19G294 were linked by short peptide (SEQ ID NO: 12), and another short peptide (SEQ ID NO: 13) was added downstream, then conjugated with the scFv (SEQ ID NO: 57) . Optimization was performed according to the CHO cell codon preference. The AvrII restriction site and kozak sequence were introduced upstream, and the stop codon and BstZ17I restriction site were added downstream. It was directly synthesized into pUC57 plasmid and named puc57-21G587.

The target gene was amplified and the PCR product was recovered by 1% agarose electrophoresis. The recovered PCR product and pZHK2.0 vector were digested by AvrII and BstZ17I. T4 ligase ligated the double digestion product into pZHK2.0 vector and transformed into Top10 competent cells. The cells were plated on LB plates containing kanamycin resistance and incubated overnight at 37°C. The next day, the positive clones were screened and sequenced. The sequence was completely consistent with the expected sequence, and the humanized CD26-CD3 BiTE expression plasmid was obtained.

The mammalian cell line with high expression of CD26-CD3 humanized BiTE was cultured in Dynamis medium , and fed batch culture was performed at 37°C, 8% CO₂ and 130rpm. The supernatant of the culture medium was centrifuged at 12000rpm for 15min to remove the cell pellet at low-temperature. And then the supernatant was filtered through a 0.45µm filter membrane, followed by chromatography purification. The target products were all about 55kDa.

### Example 8. Dot blot assay of purified humanized bispecific antibodies

The purified sample was diluted into different concentration gradients, 2µl of the diluted sample was placed on the NC membrane, and the positive control sample was 17G29. After complete absorption, the membrane was blocked with 5% skim milk solution, shaked at room temperature for 1 hour. The membrane was washed 3 times with TBST for 5min each time.

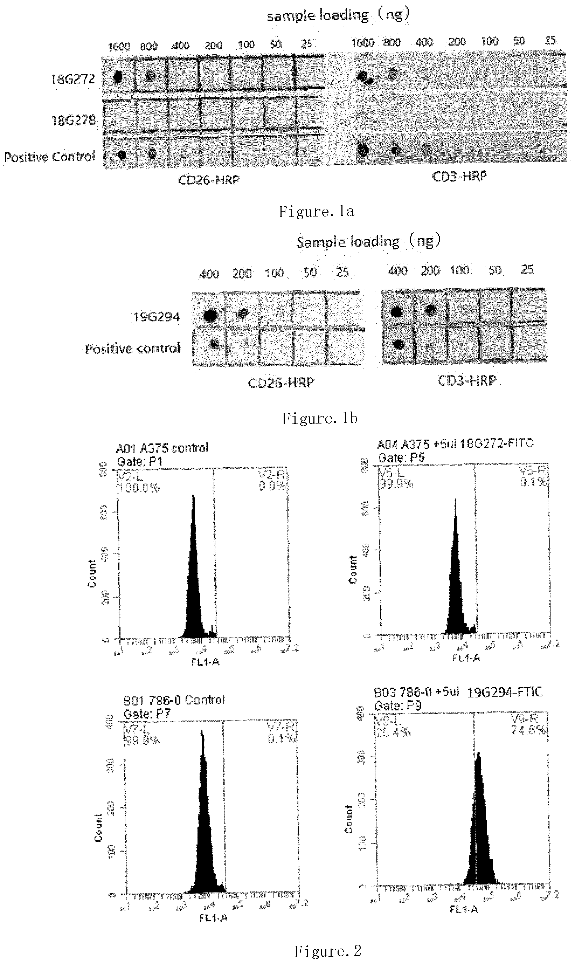

CD3-HRP protein was added to 2.5% skim milk solution at 1:1000 (50ul CD3-HRP was added to 50 ml of 2.5% skim milk solution), shaked at room temperature for 1 hour, and washed three times with TBST for 5min each time. CD26-HRP protein was added to 2.5% skim milk solution at 1:1000 (50 ul CD26-HRP was added to 50ml of 2.5% skim milk solution), shaked at room temperature for 1 h, and washed three times with TBST for 5min each time. The TBST solution on the film was blotted, and the color development solution (SuperSignal West Pico Chemiluminescent Substrate) was added. After 1min of color development, the color development solution was blotted and exposed in the gel imaging system. The continuous exposure program was selected and set to 15s/ sheet. Twelve consecutive exposures were made. Dot blot analysis of 18G272(humanized antibody derived from NO.62 mouse antibody and 18G278(humanized antibody derived from NO.212 mouse antibody) showed that the binding activity of 18G272 to CD26 and CD3 at different concentrations was close to that of the positive control 17G29(anti-CD26-CD3 humanized bispific antibody derived from YS110). It was significantly higher than that of 18G278(FIG. 1-a). Six samples of 19G292-19G297(anti-CD26-CD3 humanized bispecific antibody derived from No.62 mouse antibody) were detected by dot blot. Both 19G292-19G297 were found to bind CD26 and CD3. The binding activity of 19G292-19G296 to CD26 was similar to that of the positive control 17G29, and the binding activity of 19G294 was even better than that of the positive control (FIG. 1-b).

### Example 9 Studies on the binding specificity of the humanized bispecific antibody to the antigen of target cell

### 1. Screening of CD26-positive and CD26-negative cells

The target cells were cultured in T75 cell culture flask. When the cell fusion was more than 80%, the cells were digested by trypsin and collected, washed once with PBS, and counted by ahemocytometer. The cells were divided into 5×10⁵ cells each. Anti-CD26 monoclonal antibody was used as the primary antibody, and incubated with the target cells at room temperature for about 40min. After incubation and centrifugation, the supernatant was discarded, and the cell precipitate was resuspended in PBS. The supernatant was discarded after centrifugation again, and the cell precipitate was collected. Then Alexa Fluor 488 mouse anti-human IgG1 was used as the secondary antibody. The cells were resuspended and incubated at room temperature in a dark place for about 30min. After incubation, the cells were washed twice with PBS, the supernatant was discarded after centrifugation, and the cell precipitates were collected. The cells were resuspended in 200ul of PBS, and the positive rate of CD26 was analyzed by flow cytometer within 1h.

Identified by flow cytometer, CD26 positive cell lines were 786-0, OS-RC-2, A498, NCI-H226, NCI-H2052, NCI-H596, HCC827, Huh-7 and PC-3, and CD26 negative cell lines were G401 and A-375. Their positive rates are shown in Table 4. The CD26 positive cell line was consistent with the reference (Chinese Patent application number CN200680034937.4, CD26/DPP4-a potential biomarker and target for cancer therapy,Pharmacology&Therapeutics(2019), 198:135-159).

**Table 4 CD26 positive rate of target cells**

| **NO** | **cell name** | **tumors** | **positive rate of CD26** |
|---|---|---|---|
| 1 | 786-0 | Kidney cancer | 99.9% |
| 2 | OS-RC-2 | | 97.8% |
| 3 | A498 | | 97.1% |
| 4 | G401 | | 0% |
| 5 | NCI-H226 | Mesothelioma | 99.4% |
| 6 | NCI-H2052 | | 88.2% |
| 7 | NCI-H596 | Lung cancer | 72.1% |
| 8 | HCC 827 | | 93.8% |
| 9 | Huh-7 | Liver cancer | 95.3% |
| 10 | PC-3 | Prostate cancer | 93.8% |
| 11 | A-375 | Melanoma | 2.90% |

### 2, FITC labeled antibody:

The fluorescein FITC was weighed and dissolved in DMSO solution to a final concentration of 1mg/ml. 500ul of 18G272 antibody was added to a final concentration of 1mg/ml, and 1/10 volume of 1M Na₂CO₃ solution was added, and 15ul of FITC solution was added. Mix well. The mixture was incubated at room temperature in the dark for 3.5h. Then, the mixture was centrifuged with an ultrafiltration tube with a interception diameter of 10KDa to completely remove FITC that was not bound to 18G272, and the solution in the tube was collected.

### 3. Flow cytometry:

When the cell fusion was about 80%, the cells cultured in T75 cell culture flask were digested with trypsin and collected. The cells were resuspended in an appropriate amount of PBS, and counted with a hemacytometer, and divided into 5×10⁵ cells each. The cell precipitates were collected after centrifugation. The target cells were resuspended with FITC-labeled 18G272 solution, placed on a homogenizer, and incubated at room temperature in the dark for 30min. After incubation, the cells were resuspended in PBS, the supernatant was discarded after centrifugation, and the operation was repeated once. Cell precipitates were resuspended in 500ul of PBS solution. Negative control: In another sample, no antibody sample was added in the labeling process, the remaining steps were performed in parallel with the above process, and the resulting solution was the FITC-labeled negative control. A BD Accuri C6 flow cytometer was used to analyze the positive rate of FITC labeling.

The results showed that the humanized bispecific antibody 18G272 could bind to CD26 positive cells , such as 786-0 cells, NCI-H226 cells, PC-3 cells and HCC 827 cells, but not to CD26 negative A375 cells. Its binding to the CD26 target is specific.

The same method was used to detect the binding specificity of the humanized bispecific antibody 19G294 to the target cell antigen. 19G294 could bind to CD26 positive cells , such as 786-0 cells, NCI-H226 cells, PC-3 cells, OS-RC-2 cells, but not to CD26 negative G401 cells. The humanized bispecific antibody 19G294 could bind to CD26 positive cells but not to CD26 negative cells. Its binding to the CD26 target is specific.

Representative figures of binding specificity of antibody to target cell antigens determined by flow cytometry were shown in Figure 2, and detailed data were shown in Tables 5 and 6.

**Table 5 Binding specificity of antibody 18G272 to target cell antigen determined by flow cytometry**

| Cells name | 786-0 | NCI-H226 | PC-3 | HCC 827 | A375 |
|---|---|---|---|---|---|
| The rate of specific binding | 74.6% | 80.7% | 64.9% | 41.7% | 0.1% |

**Table 6 Binding specificity of antibody 19G294 to target cell antigen determined by flow cytometry**

| Cells name | 786-0 | NCI-H226 | NCI-H2052 | PC-3 | OS-RC-2 | G401 |
|---|---|---|---|---|---|---|
| The rate of specific binding | 79.0% | 79.5% | 70.4% | 50.6% | 91.6% | 0.0% |

Other humanized bispecific antibodies of the present invention also bind to CD26 positive cells, but not to CD26 negative cells, and have specific binding to cells with CD26 antigen.

### Example 10 Affinity analysis of humanized bispecific antibody with CD26 protein and CD3 protein

### 1, affinity study with CD26 protein

CD26 protein was biotinylated with EZ-link NHS-PEG12-Biotin. Biotinylated CD26 protein was immobilized to SA biosensor. Equilibrated in 1×Kinetics solution. The CD26 immobilized to SA biosensor was combined with the the antibody solution to be detected. The concentration gradients of the antibody solution were 31.3nM, 62.5nM, 125nM, 250nM, and 500nM. The biosensor was dissociated in 1×Kinetics solution. Fortebio Data Analysis 8.0 software was used for data analysis and affinity constant values were calculated.

**Table 7 Affinity of humanized bispecific antibody to CD26 protein**

| Sample | KD(M) | kon (1/Ms) | kdis(1/s) |
|---|---|---|---|
| 19G294 | 0.914E-09 | 9.24E+04 | 8.45E-05 |
| 19G292 | <1.0E-12 | 6.58E+04 | No dissociation |
| 19G293 | <1.0E-12 | 5.46E+04 | No dissociation |
| 19G296 | 1.48E-08 | 9.45E+04 | 1.40E-03 |
| 19G297 | 4.40E-09 | 9.18E+04 | 3.70E-04 |
| 17G29 | 1.18E-09 | 1.83E+05 | 2.16E-04 |
| 21G587 | 0.352E-09 | 1.14E+05 | 4.01E-05 |

According to the above data, when the anti-CD3 segment was the same, the affinity constant of the humanized antibodies (19G292, 19G293, 19G294) derived from NO.62 mouse antibody was lower than that of the humanized antibodies derived from NO.212 mouse antibody or YS 110, indicating that the former had a higher affinity to CD26. In addition, 19G294 and 21G587 had the same anti-CD26 segment but different anti-CD3 segment, and both had the same level of affinity with CD26 protein.

### 2. Affinity of antibody to CD3 protein

CD3 protein was biotinylated with EZ-link NHS-PEG12-Biotin. Biotinylated CD3 protein was immobilized to SA biosensor. Equilibrated in 1×Kinetics solution. The CD3 immobilized to SA biosensor was combined with the the humanized bispecific antibody solution to be detected.The concentration gradient of humanized bispecific antibody was 200nM, 400nM, 800nM, and 1600nM. The biosensor was dissociated in 1×Kinetics solution. Fortebio Data Analysis 8.0 software was used for data analysis and affinity constant values were calculated.

**Table 8 Affinity of humanized bispecific antibody to CD3 protein**

| Sample name | KD(M) | kon (1/Ms) | kdis(1/s) |
|---|---|---|---|
| 19G294 | 0.751 E-09 | 2.38E+05 | 1.78E-04 |
| 19G292 | 0.568 E-09 | 1.88E+05 | 1.07E-04 |
| 19G293 | 1.73 E-09 | 3.31E+05 | 5.71E-04 |
| 19G296 | 9.67E-09 | 2.33E+05 | 2.25E-03 |
| 19G297 | 1.01E-08 | 3.62E+05 | 3.67E-03 |
| 17G29 | 12.2 E-09 | 9.08E+04 | 5.01E-05 |
| 21G587 | 0.683 E-09 | 1.28E+05 | 8.71E-05 |

In this experiment, the affinity constant of 19G294 to CD3 protein was 0.751nM. After binding to CD3, 19G296 and 19G297 dissociated rapidly. With the same anti-CD3 segment, the affinity of humanized bispecific antibodies 19G294, 19G292 and 19G293 derived from No. 62 mouse antibody to CD3 protein was significantly higher than that of humanized bispecific antibody 17G29 derived from YS110. In addition, 19G294 and 21G587 have the same anti-CD26 segment but different anti-CD3 segment, and both have the same level of affinity with CD3 protein.

Combined with the affinity to CD26 protein (Table 7) and CD3 protein (Table 8) of the above antibodies, the humanized antibodies derived from No.62 mouse antibodies (19G292, 19G293, 19G294) showed better CD26 protein binding ability than the humanized antibodies derived from No.212 mouse antibodies (19G296, 19G297). The humanized antibodies (19G292, 19G293, 19G294) derived from No.62 mouse antibodies showed better CD3 binding and dissociation ability than those derived from No.212 mouse antibodies (19G296, 19G297) and 17G29 humanized antibodies derived from YS110. 19G294 is a more advantageous combination of bispecific antibody molecules.

### 3. Observation of simultaneously binding to CD26 protein and CD3 protein of bispecific antibody

The 19G294 protein was biotinylated with EZ-link NHS-PEG12-Biotin. The biotinylated 19G294 protein was immobilized to SA biosensor. Equilibrated in 1×Kinetics solution; The antibody ( 19G294 protein ) immobilized to SA biosensor was first combined with 400nM CD26 protein solution, and then placed in 400nM CD26 solution containing 400nM CD3 protein. Or, the antibody ( 19G294 protein ) immobilized to SA biosensor was first combined with 400nM CD3 protein solution, and then placed in 400nM CD3 solution containing 400nM CD26 protein . Fortebio Data Analysis 8.0 software was used to observe the binding.

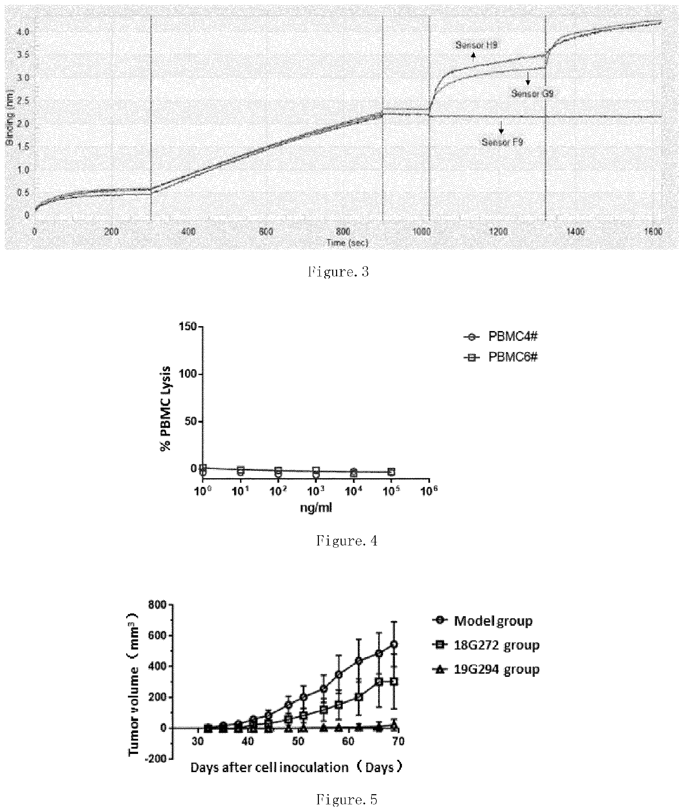

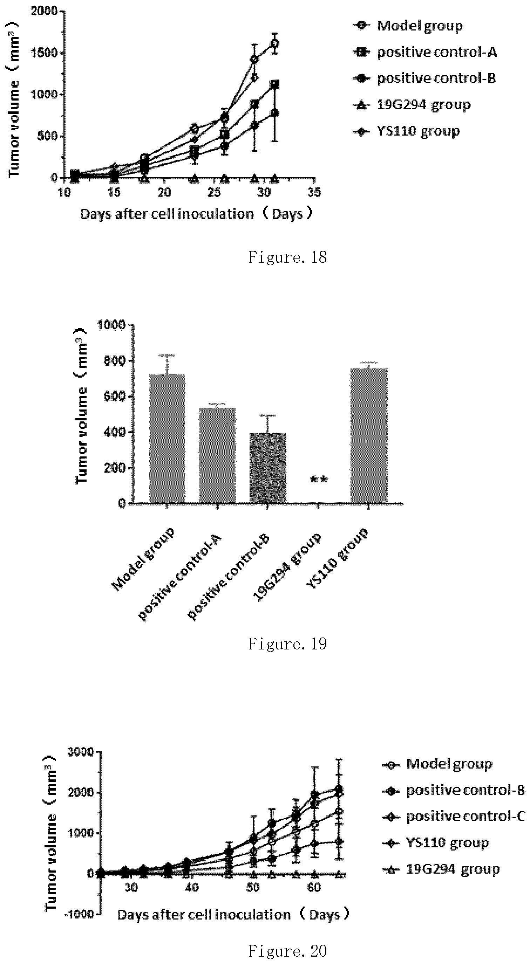

As shown in Figure 3, 19G294 was fixed with SA sensor.After saturated binding to CD26, 19G294 could continue to bind to CD3 protein in CD26 solution (Sensor G9 curve shown in Figure 3). After saturated binding to CD3 , 19G294 can continue to bind to CD26 protein in CD3 solution (Sensor H9 curve shown in FIG. 3). Sensor F9 in FIG. 3 was the negative control proving that no nonspecific binding of the sensor to the solution occurred. The results showed that 19G294 could bind to CD26 or CD3 protein first, and then could still bind to CD3 or CD26 protein. There was no sequence of binding.

### Example 11 Evaluation of humanized bispecific antibody mediated cytotoxicity of PBMC to target cells

### 1, 786-0 cell ( human renal cancer cell ) model

In the 786-0 cell reaction system, 786-0 cells were labeled with green fluorescent signal by fluorescent dye Calcein-AM. The 786-0 cells were seeded into U-type 96-well cell culture plate at a cell concentration of 6×10⁵cells/ml, 50µl per well. 50µl of humanized bispecific antibody with final concentrations of 100ng/ml, lOng/ml, 1ng/ml, 0.1ng/ml, 0.01ng/ml and 0.001ng/ml were added to the corresponding sample reaction wells. 50µl of culture medium was added to the blank control wells. Add 50 µl of 1% TritonX-100 to the positive control well. Then add 50µl of PBMC cells with a concentration of 6×10⁶cells/ml at the E/T ratio of 10:1, and incubate the reaction system at 37°C in carbon dioxide for 5h. After the reaction and centrifugation, the supernatant was placed in a new 96-well plate, and after centrifugation again, 80µl of the supernatant was placed in a black 96-well plate for detection at 470nm excitation wavelength and 515nm emission wavelength using a microplate reader.

### 2, OS-RC-2 cell ( human renal cancer cell ) model

In OS-RC-2 cell reaction system, OS-RC-2 cells were labeled with green fluorescent signal by fluorescent dye Calcein-AM. OS-RC-2 cells were seeded into U-type 96-well cell culture plate at a cell concentration of 6×10⁵cells/ml, 50µl per well. 50µl of humanized bispecific antibody with final concentrations of 100ng/ml, lOng/ml, 1ng/ml, 0.1ng/ml, 0.01ng/ml and 0.001ng/ml were added to the corresponding sample reaction wells.50µl of culture medium was added to the blank control wells. Add 50µl of 1% TritonX-100 to the positive control well. Then add 50 µl of PBMC cells with a concentration of 9×10⁶cells/ml at the E/T ratio of 15:1, and incubate the reaction system at 37°C in carbon dioxide for 5h. After the reaction and centrifugation, the supernatant was placed in a new 96-well plate, and after centrifugation again, 80µl of the supernatant was placed in a black 96-well plate for detection at 470nm excitation wavelength and 515nm emission wavelength using a microplate reader.

### 3, NCI-H226 cell ( human mesothelioma ) model

In the NCI-H226 cell reaction system, NCI-H226 cells were labeled with green fluorescent signal by fluorescent dye Calcein-AM, and NCI-H226 cells were seeded into U-type 96-well cell culture plate at a cell concentration of 6×10⁵cells/ml, 50µl per well. 50µl of humanized bispecific antibody with final concentrations of 100ng/ml, lOng/ml, 1ng/ml, 0.1ng/ml, 0.01ng/ml and 0.001ng/ml were added to the corresponding sample reaction wells. 50µl of culture medium was added to the blank control wells. Add 50µl of 1% TritonX-100 to the positive control well. Then add 50 µl of PBMC cells with a concentration of 9×10⁶cells/ml at the E/T ratio of 15:1, and incubate the reaction system at 37°C in carbon dioxide for 5h. After the reaction and centrifugation, the supernatant was placed in a new 96-well plate, and after centrifugation again, 80µl of the supernatant was placed in a black 96-well plate for detection at 470nm excitation wavelength and 515nm emission wavelength using a microplate reader.

### 4, NCI-H2052 cell ( human mesothelioma ) model

In the NCI-H2052 cell reaction system, NCI-H2052 cells were labeled with green fluorescent signal by fluorescent dye Calcein-AM, and NCI-H2052 cells were seeded into U-type 96-well cell culture plate at a cell concentration of 6×10⁵cells/ml, 50µl per well. 50µl of humanized bispecific antibody with final concentrations of 100ng/ml, lOng/ml, 1ng/ml, 0.1ng/ml, 0.01ng/ml and 0.001ng/ml were added to the corresponding sample reaction wells. 50µl of culture medium was added to the blank control wells. Add 50µl of 1% TritonX-100 to the positive control well. Then add 50 µl of PBMC cells with a concentration of 9×10⁶cells/ml at the E/T ratio of 15:1, and incubate the reaction system at 37°C in carbon dioxide for 5h. After the reaction and centrifugation, the supernatant was placed in a new 96-well plate. After centrifugation again, 80µl of the supernatant was placed in a black 96-well enzyme plate and detected with a microplate reader at 470nm excitation wavelength and 515nm emission wavelength.

### 5, PC-3 cell ( human prostate cancer ) model

In the PC-3 cell reaction system, PC-3 cells were labeled with green fluorescent signal by fluorescent dye Calcein-AM. PC-3 cells were seeded into U-type 96-well cell culture plate at a cell concentration of 6×10⁵cells/ml, 50µl per well. 50µl of humanized bispecific antibody with final concentrations of 100ng/ml, lOng/ml, 1ng/ml, 0. 1ng/ml, 0.01ng/ml and 0.001ng/ml were added to the corresponding sample reaction wells. 50µl of culture medium was added to the blank control wells. Add 50 µl of 1% TritonX-100 to the positive control well. Then add 50µl of PBMC cells with a concentration of 9×10⁶cells/ml at the E/T ratio of 15:1, and incubate the reaction system at 37°C in carbon dioxide for 5h. After the reaction and centrifugation, the supernatant was placed in a new 96-well plate, and after centrifugation again, 80µl of the supernatant was placed in a black 96-well plate for detection at 470nm excitation wavelength and 515nm emission wavelength using a microplate reader.

### 6, HCC 827 cell ( human non-small cell lung cancer ) model

In the HCC 827 cell reaction system, HCC 827 cells were labeled with green fluorescent signal by fluorescent dye Calcein-AM. HCC 827 cells were seeded into U-type 96-well cell culture plate at a cell concentration of 6×10⁵cells/ml, 50µl per well. 50µl of humanized bispecific antibody with final concentrations of 100ng/ml, lOng/ml, 1ng/ml, 0.1ng/ml, 0.01ng/ml and 0.001ng/ml were added to the corresponding sample reaction wells.50µl of culture medium was added to the blank control wells. Add 50µl of 1% TritonX-100 to the positive control well. Then add 50 µl of PBMC cells with a concentration of 6×10⁶cells/ml at the E/T ratio of 10:1, and incubate the reaction system at 37°C in carbon dioxide for 5h. After the reaction and centrifugation, the supernatant was placed in a new 96-well plate. After centrifugation again, 80µl of the supernatant was placed in a black 96-well enzyme plate and detected with a microplate reader at 470nm excitation wavelength and 515nm emission wavelength.

### 7. Experimental results

Cell lysis rate = (Vₛₐₘₚₗₑ-V_{vehicle control})/(V_{TritonX-100}-V_{vehicle control})× 100%. (Vₛₐₘₚₗₑ is the mean value of the fluorescence signal reading of the drug treatment group, V_{vehicle control} is the mean value of the fluorescence signal reading of the blank control group, and V_{Triton-100} is the mean value of the fluorescence signal reading of the positive control group.)

Cell lysis rate and sample concentration values were used to calculate the IC₅₀ value of samples mediated PBMC cytotoxicity against target cells using GraphPad Prism 7.00 software.

**Table 9 IC₅₀ values of different humanized bispecific antibodies mediated PBMC cytotoxicity against different target cells (pg/ml)**

| Cell name | 18G272 | 19G292 | 19G293 | 19G294 | 19G295 | 19G296 | 19G297 | 17G29 | YS110 |
|---|---|---|---|---|---|---|---|---|---|
| 786-0 | 2275 | 805.5 | 974 | 260.0 | inactive | inactive | inactive | 1034 | >100ng/ml |
| OS-RC-2 | 283.7 | 1043 | 2246 | 544.9 | inactive | inactive | inactive | 958.1 | >100ng/ml |
| NCI-H226 | 142.8 | 1102 | 404.6 | 752.3 | inactive | inactive | inactive | 917.6 | >100ng/ml |
| PC-3 | 378.8 | 339.2 | 564.9 | 219.6 | inactive | inactive | inactive | 1140 | >100ng/ml |
| HCC 827 | - | - | - | 546.4 | - | - | - | - | - |
| NCI-H2052 | - | - | - | 5053 | - | - | - | - | - |

| | | | | | | | | | |
|---|---|---|---|---|---|---|---|---|---|
| - : means no testing. | | | | | | | | | |

Anti-CD26-CD3 humanized bispecific antibodies derived from No. 62 mouse antibody, such as 18G272, 19G292, 19G293 and 19G294, can mediate PBMC cytotoxicity against a variety of CD26 positive tumor cells, and had killing activity in vitro. Anti-CD26-CD3 humanized bispecific antibodies such as 19G295, 19G296 and 19G297 derived from No. 212 mouse antibody did not mediate PBMC cytotoxicity against CD26 positive tumor cells.

On the one hand, the mouse antibody screened in this application have better killing activity against CD26 positive tumor cells. The anti-CD26-CD3 humanized antibody derived from No.62 mouse antibody had better killing effect on a variety of CD26 positive tumors than 17G29 (the anti-CD26-CD3 humanized antibody derived from No.62 mouse antibody ) and the anti-CD26-CD3 humanized antibody derived from No.212 mouse antibody. On the other hand, the BiTE (18G272, 19G292, 19G293, 19G294, 17G29) presented in this study showed better killing effects on a variety of CD26 positive tumor cells than the full-length IgG antibody YS110.

### Example 12 Activation of PBMC cells by a humanized bispecific antibody in the process of mediating the cytotoxic effect of PBMC on target cells

PBMC cells + bispecific antibody system: PBMC cells were collected, counted, resuspended to 1×10⁶cells/ml, 200ul per well was added to 12-well cell culture plates, antibody was diluted to a final concentration of 1ng/ml, and co-incubated for 24 hours. PBMC cell + bispecific antibody +OS-RC-2 cell system: PBMC cells were collected, counted, resuspended to 6×10⁵cells/ml, 100ul/well was added to 12-well cell culture plates, antibody was diluted to a final concentration of 1ng/ml, OS-RC-2 cells were diluted to 9×10⁶cells/ml, 100ul per well was added to the plates, co-incubated for 24h.

After incubation and centrifugation, the cell precipitate was collected , resuspended in PBS, added anti-CD25-APC antibody (purchased from Miltenyl Company) and anti-CD69-PE antibody (purchased from Miltenyl Company), incubated at 4°C for 10min, and washed twice with PBS. After resuspension with PBS, the samples were analyzed by BD ACCURI C6 flow cytometer.

In the "PBMC + bispecific antibody" system, bispecific antibody did not induce the upregulation of CD25 and CD69 in PBMC. In the system of "PBMC + bispecific antibody +OS-RC-2", bispecific antibody induced the upregulation of CD25 and CD69 in PBMC and promoted the activation of PBMC. In the system of "PBMC +YS110+OS-RC-2 ", YS110 did not induce the upregulation of CD25 and CD69 expression in PBMC. See Table 10.

**Table 10 The expression of CD25 and CD69 in PBMC during antibody-mediated cytotoxic effect on target cells determined by flow cytometry**

| reaction system | PBMC | PBMC+ 18G272 | PBMC+ 19G294 | PBMC+ 18G272+OS-RC-2 | PBMC+ 19G294+OS-RC-2 | PBMC+YS110 +OS-RC-2 |
|---|---|---|---|---|---|---|
| expression level of CD25 | 2.4% | 5.0% | 3.5% | 23.7% | 23.5% | 2.1% |
| expression level of CD69 | 5.2% | 10.3% | 9.8% | 55.5% | 58.9% | 4.7% |

The same phenomenon was also observed in other CD26-positive tumor cells such as 786-0. In the "PBMC + bispecific antibody " system, bispecific antibody did not induce the upregulation of CD25 and CD69 expression in PBMC cells. The expression of CD25 and CD69 in PBMC cells was induced by "PBMC cells + bispecific antibody + other CD26 positive tumor cells (786-0, etc.)".

CD25 and CD69 are important markers of T cell activation. The above results demonstrated that the full-length IgG antibody YS 110 does not effectively induce T cell activation. CD26-CD3 bispecific antibody does not cause significant T cell activation before reaching the tumor. Bispecific antibody can cause T cells activation around the tumor after binding to turner cells, and then kill the tumor cells, and improves the safety of the action.

### Example 13 Cytokine detection in the process of humanized bispcific antibody mediated cytotoxic effect of PBMC on target cells

OS-RC-2 cells were resuspended to 6×10⁵cells/ml, and 100µl per well was added to 12-well plate. Single donor derived PBMC cells were collected and resuspended to 9×10⁶cells/ml, 100µl was added to each well, and 100µl of 19G294, 18G272 and 17G29 samples with concentrations of 0ng/ml, 3ng/ml were added respectively, and the final volume was 300µl. The reaction system was incubated at 37 °C for 2h, 4h, 6h, and 21h, and the culture supernatant was collected. After centrifugation, the supernatant was taken for ELISA detection. The levels of IFN-γ, TNF-α, L-4 and IL-10 were detected by ELISA kit.

ELISA kits were used to detect the cytokine content of each sample as shown in the table below.

**Table 11 IFN-γ content detected by ELISA (pg/ml)**

| | 2h | 4h | 6h | 21h |
|---|---|---|---|---|
| 0ng/ml | -7.65 | 143.7 | -58.3 | 37.7 |
| 18G272 | 116.7 | 667.7 | 964.7 | 1181.7 |
| 19G294 | 91.7 | 337.7 | 303.7 | 398.7 |
| 17G29 | 341.7 | 1395.7 | 1777.7 | 2368.7 |

**Table 12 TNF-α content detected by ELISA (pg/ml)**

| | 2h | 4h | 6h | 21h |
|---|---|---|---|---|
| 0ng/ml | 16.01 | 19.55 | 17.56 | 10.33 |
| 18G272 | 161.3 | OVERFLOW* | 192.51 | 158.73 |
| 19G294 | 111.88 | 166.88 | 111.79 | 47.22 |
| 17G29 | 165.43 | 182.13 | 183.73 | 180.33 |

**Table 13 IL-10 content detected by ELISA (pg/ml)**

| | 2h | 4h | 6h | 21h |
|---|---|---|---|---|
| 0ng/ml | 8.4 | 16.5 | 19.3 | 28.9 |
| 18G272 | 15.9 | 53.7 | 86.2 | 190.6 |
| 19G294 | 9.9 | 32.3 | 47.3 | 65.9 |
| 17G29 | 22.8 | 113.1 | 90.2 | 200.4 |

**Table 14 IL-4 content detected by ELISA (pg/ml)**

| | 2h | 4h | 6h | 21h |
|---|---|---|---|---|
| 0ng/ml | 3.56 | 4.08 | 3.44 | 3.05 |
| 18G272 | 7.67 | 19.21 | 21.64 | 6.64 |
| 19G294 | 3.69 | 10.74 | 11.38 | 3.69 |
| 17G29 | 23.82 | 58.69 | 57.79 | 43.44 |

| | | | | |
|---|---|---|---|---|
| * indicates that the reading value is too high to exceed the device detection limit. | | | | |

In summary, in the process of humanized bispecific antibody-mediated killing effect of PBMC on target cells, the cytokines secreting of PBMC introduced by anti-CD26-CD3 humanized bispecific antibodies 19G294 and 18G272 derived from No. 62 mouse antibody were lower than those induced by anti-CD26-CD3 humanized bispecific antibodies 17G29 derived from YS110 at all time points with the same dosage. For this type of molecular structure targeting CD3 and activating T cells, low cytokine storm caused by cytokine secretion of T cells is associated with higher safety. (A FAD oncology analysis of CD3 bispecific constructs and first-in-human dose selection(2017), Regulatory Toxicology and Pharmacology, 90:144-152.). The bispecific antibodies 19G294 and 18G272 of this application had the advantage of inducing lower cytokines secreting of T cells, which may be related to recognizing different CD26 epitopes with 17G29. Compared with other epitopes, the antigen epitope recognized by the proposed antibody is related to reducing the risk of cytokine storm and improving safety.

### Example 14 Humanized bispecific antibody mediated toxicity of PBMC to PBMC

### 1, 786-0 cell model

PBMC cells were labeled with green fluorescent signal by fluorescent dye Calcein-AM. 786-0 cells were seeded into U-type 96-well cell culture plate at a cell concentration of 6×10⁵cells/ml, 50µl per well. 50µl of humanized bispecific antibody with final concentrations of 100ng/ml, lOng/ml, 1ng/ml, 0.1ng/ml, 0.01ng/ml and 0.001ng/ml were added to the corresponding sample reaction wells. 50µl of culture medium was added to the blank control wells. 50µl TritonX-100 (final concentration 1%) was added to the positive control well. Then 50µl PBMC cells with concentration of 9×10⁶cells/ml were added at the E/T ratio of 10:1. The reaction system was incubated at 37°C in carbon dioxide for 5h. After the reaction and centrifugation, the supernatant was placed in a new 96-well plate. After centrifugation again, 80µl of the supernatant was placed in a black 96-well enzyme plate and detected using a microplate reader at 470nm excitation light wavelength and 515nm emission wavelength.

### 2, OS-RC-2 cell model

PBMC cells were labeled with green fluorescent signal by fluorescent dye Calcein-AM. OS-RC-2 cells were seeded into U-type 96-well cell culture plate at a cell concentration of 6×10⁵cells/ml, 50µl per well. 50µl of humanized bispecific antibody 18G272 with final concentrations of 100ng/ml, lOng/ml, 1ng/ml, 0.1ng/ml, 0.01ng/ml and 0.001ng/ml were added to the corresponding sample reaction wells. 50µl of culture medium was added to the blank control wells. 50µl TritonX-100 (final concentration of 1%) was added to the positive control well. Then 50µl PBMC cells with concentration of 9×10⁶cells/ml were added at the E/T ratio of 15: 1. The reaction system was incubated at 37°C in carbon dioxide for 5h. After the reaction and centrifugation, the supernatant was placed in a new 96-well plate. After centrifugation again, 80µl of the supernatant was placed in a black 96-well enzyme plate and detected using a microplate reader at 470nm excitation light wavelength and 515nm emission wavelength.

### Analysis of the results

Cell lysis rate = (Vₛₐₘₚₗₑ-V_{vehicle control})/(V_{TritonX-100}-V_{vehicle control})× 100%. (Vₛₐₘₚₗₑ is the mean value of the fluorescence signal reading of the drug treatment group, V_{vehicle control} is the mean value of the fluorescence signal reading of the solvent control group, and V_{Triton-100} is the mean value of the fluorescence signal reading of the positive control group.) The IC₅₀ values of each sample mediated toxicity of PBMC to PBMC were calculated by GraphPad Prism 7.00 software.

In the process of PBMC killing 786-0 mediated by antibody 18G272, the ability of PBMC to kill PBMC cells was very low. The IC₅₀ value of 18G272-mediated cytotoxicity of PBMC(4#) against PBMC(4#) cells was 99730000pg/ml. PBMC(6#) had no 18G272-mediated cytotoxic effect on PBMC(6#) cells. The results are shown in FIG. 4.

In the process of PBMC killing OS-RC-2 mediated by antibody 18G272, 18G272 did not mediate killing effect of PBMC to PBMC cells. PBMC(1#) had no 18G272-mediated cytotoxic effect on PBMC(1#) cells. PBMC(3#) had no 18G272-mediated cytotoxic effect on PBMC(3#) cells.

In the process of PBMC killing 786-0 mediated by antibody 19G294, the ability of PBMC to kill PBMC cells was very low. The IC₅₀ value of 19G294-mediated cytotoxicity of PBMC(2#) against PBMC(2#) cells was 1915313pg/ml. The IC₅₀ value of 19G294-mediated cytotoxicity of PBMC(3#) against PBMC(3#) cells was 1947224pg/ml.

In the process of PBMC killing OS-RC-2 mediated by antibody 19G294, the ability of PBMC to kill PBMC cells was very low. The IC₅₀ value of 19G294-mediated cytotoxicity of PBMC(2#) against PBMC(2#) cells was 47920315pg/ml. The IC₅₀ value of 19G294-mediated cytotoxicity of PBMC(3#) against PBMC(3#) cells was 1406115138pg/ml.

In summary, CD26-CD3 humanized bispecific antibody can accurately recognize PBMC. Although it has been reported that T cells express CD26, the bispecific antibodies 18G272 and 19G294 in this application do not mediate cytotoxicity of PBMC cells to PBMC cells obviously, which proved that it has good safety.

### Example 15 In vitro efficacy comparison of 19G294 and drug B (BAVENCIO, PD-L1 antibody)

The target cells were cultured in T75 cell culture flask. When the cell fusion was more than 80%, the cells were digested by trypsin and collected, washed once with PBS, and counted by a hemocyte counter, and divided into 5×10⁵ cells each. The target cells were incubated with PE-labeled anti-PD-L1 monoclonal antibody (purchased from Sino Biological Company) for about 40min at room temperature in the dark. After incubation and centrifugation, the supernatant was discarded, the cell precipitate was resuspended in PBS, the supernatant was discarded after centrifugation again, the cell precipitate was collected, and resuspended in about 200ul of PBS solution. The positive rate of PD-L1 was detected by flow cytometry within 1 hour.

By flow cytometry, the PD-L1 positive rate was 66.2% in 786-0 cell line and 29.6% in A498 cell line.

**Table 15 Positive rate detection of PD-L1**

| | 786-0 | A498 |
|---|---|---|
| PD-L1 | 66.2% | 29.6% |

Human renal carcinoma 786-0-luc cells (the Luciference reporter gene was transfected into 786-0 cells) and human renal carcinoma A498-luc cells (the Luciference reporter gene was transfected into A498 cells) were seeded into white bottom opaque 96-well cell culture plates at a concentration of 3×10⁵cells/ml, 50µl per well. 50µl of antibody at final concentrations of 100ng/ml, lOng/ml, 1ng/ml, 0.1ng/ml, 0.01ng/ml, and 0.001ng/ml was added to the corresponding reaction wells, respectively. Then 50µl PBMC cells with a concentration of 3×10⁶cells/ml were added at the E/T ratio of 10: 1.The reaction system was incubated at 37°C in carbon dioxide for 21h. After the reaction, the reaction system was centrifuged, the supernatant was discarded, and 100ul reporter gene detection reagent (purchased from Vazyme Company) was added. After shaking at 400rpm for 5min on a microplate oscillator, the assay was performed immediately with a microplate reader under Lum conditions. GraphPad Prism 7.00 software was used to make a 4-parameter curve according to the reduced level of reporter gene and the dose of antibody, and calculated the EC₅₀ value . The results were shown in Table 16.

**Table 16 Antibody mediated cytotoxicity of PBMC against target cells (EC₅₀pg/ml)**

| | 786-0-LUC | A498-LUC |
|---|---|---|
| 19G294 | 2623 | 3211 |
| BAVENCIO | 1791039 | 3289316 |

According to the above data, the EC₅₀ value of 19G294 mediated PBMC cytotoxicity against two target cells was much lower than that of BAVENCIO.The expression rates of PD-L1 in 786-0 and A498 were 66.2% and 29.6%, respectively. 19G294 was significantly more active than BAVENCIO regardless of the level of PD-L1 expression.

### Example 16 In vitro efficacy comparison of 21G587 and 19G294

Human renal carcinoma 786-0-luc cells (the Luciference reporter gene was transfected into 786-0 cells) and human renal carcinoma A498-Luc cells (the Luciference reporter gene was transfected into A498 cells) were seeded into white bottom opaque 96-well cell culture plates at a concentration of 3×10⁵cells/ml, 50µl per well. 50µl of antibody at final concentrations of 100ng/ml, lOng/ml, 1ng/ml, 0. 1ng/ml, 0.01ng/ml, and 0.00 1ng/ml was added to the corresponding reaction wells, respectively. Then 50µl PBMC cells with a concentration of 3×10⁶cells/ml were added at the E/T ratio of 10: 1.The reaction system was incubated at 37°C in carbon dioxide for 21h. After the reaction, the reaction system was centrifuged, the supernatant was discarded, and 100ul reporter gene detection reagent (purchased from Vazyme Company) was added. After shaking at 400rpm for 5min on a microplate oscillator, the assay was performed immediately with a microplate reader under Lum conditions. GraphPad Prism 7.00 software was used to make a 4-parameter curve according to the reduced level of reporter gene and the dose of antibody, and calculated the EC₅₀ value. The results were shown in Table 17.

**Table 17 Antibody-mediated cytotoxicity of PBMC against target cells (EC₅₀pg/ml)**

| | 786-0-LUC | A498-LUC |
|---|---|---|
| 21G587 | 2493 | 5397 |
| 19G294 | 1428 | 4153 |

From the above data, it can be seen that when the anti-CD26 antibody fragment of the patent was combined with different anti-CD3 fragment, 19G294 and 21G587 had the same anti-CD26 fragment and different anti-CD3 fragments, and both had the same level of in vitro efficacy.

### Example 17 Efficacy of humanized bispecific antibodies 18G272 and 19G294 in NOD/SCID mice subcutaneously bearing PC-3 human prostate cancer cell xenografts

Methods: 5×10⁶cells/0.1ml PC-3 cell suspension and 1×10⁷cells/0.1ml PBMC cell suspension were fully mixed in a volume ratio of 1:1 and inoculated subcutaneously into NOD/SCID mice aged 5 to 7 weeks weighing 18 to 22 g, 0.2mL per mouse. According to the body weight, the animals were randomly divided into three groups: Model group (PBS), 19G294 group (30µg per mouse once daily), 18G272 group (30µg per mouse once daily), with 6 animals in each group. Intravenous treatment with humanized bispecific antibodies ( 18G272 and 19G294 ) or the vehicle (PBS) started on the day of inoculation, continuously for 5 days. Two days after drug withdrawal, a second course of treatment was performed. The first treatment was 1h after inoculation, that is, the first courses were D1, D2, D3, D4, D5. The second courses were D8, D9, D10, D11 and D12. The frequency of treatment was once a day.

General clinical observation: General clinical observations were conducted at least once daily during the quarantine period and trial period, including the tumor growth and the effect of treatment on normal behavior of animals, including the death or near death, mental status, animal behaviour and other abnormal behaviour of tumor-bearing mice.

### Body weight: Body weight was conducted twice or thrice a week.

Tumor volume: Tumor volume was conducted twice or thrice a week. The length and width of the tumor were measured by vernier caliper. Tumor volume: V= (length × width²)/2. Tumor Growth Inhibition value: TGI(%) = (1-T/C) × 100%. It was generally accepted that T represented the relative tumor volume (the ratio of the tumor volume at the time of measurement to the tumor volume at the time of grouping) in the administration group and C represented the relative tumor volume (the ratio of the tumor volume at the time of measurement to the tumor volume at the time of grouping) in the model group. However, mice in this trial were divided into groups according to the body weight after inoculation. The TGI was calculated according to the T and C that represented the actual tumor volume of the treatment group and control group.

Tumor weight: Mice were euthanized at the end of the experiment, the tumor mass was stripped, rinsed with normal saline and blotted dry with filter paper, weighed and photographed. Tumor Growth Inhibition value: TGI(%)=(1-T_{TW}/C_{TW})× 100%, T_{TW} represented average tumor weight of treatment group, C_{TW} represented average tumor weight of control group.

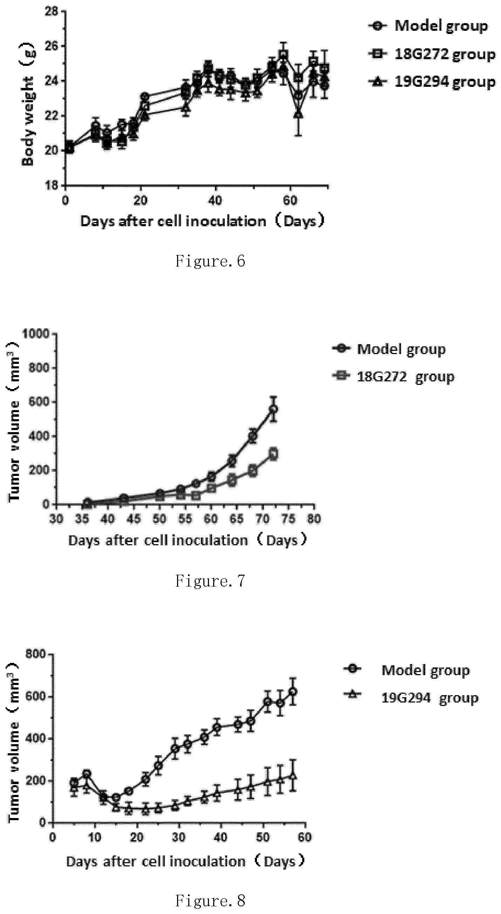

Conclusion: 18G272 had a tendency to inhibit the tumor growth of mice bearing PC-3 ( human prostate cancer ) at the dose of 30µg per mouse, and 19G294 can almost completely inhibit the tumor growth of mice bearing PC-3 . These results were shown in FIG. 5 and Table 18. At the dose of 30µg/mouse, the body weight of mice showed an upward trend in 19G294 and 18G272 groups, which was similar to that in the model group (control group), and the animals were well tolerated during the treatment. The results are shown in Figure 6 and Table 18.

**Table 18 Tumor growth and body weight of mice subcutaneously bearing PC-3**

| Groups | Tumor volume on D69 (mm³) | Tumor weight on D69 (mg) | Body weight on D1 (g) | Body weight on D69 (g) |
|---|---|---|---|---|
| Model group | 546.05±146.04 | 473.88±127.65 | 20.23±0.37 | 23.75±0.72 |
| 18G272 group | 305.82±177.85 | 225.78±120.25 | 20.22±0.34 | 24.77±1.01 |
| 19G294 group | 23.69±15.13** | 11.28±7.63** | 20.20±0.28 | 24.27±0.56 |

| | | | | |
|---|---|---|---|---|
| Note:* : P < 0.05, vs Model group; ** : P < 0.01, vs Model group. | | | | |

### Example 18 Efficacy of humanized bispecific antibody 18G272 in NOD/SCID mice subcutaneously bearing NCI-H596 human lung cancer cell xenografts

Methods: 8×10⁶cells/0. 1ml NCI-H596 cell suspension and 1.6×10⁷ cells/0.1ml PBMC cell suspension were fully mixed in a volume ratio of 1:1 and inoculated subcutaneously into NOD/SCID mice aged 5 to 7 weeks weighing 18 to 22 g, 0.2mL per mouse.According to the body weight, the animals were randomly divided into two groups: Model group (PBS, 6 mice/group) and 18G272 group (30µg per mouse once daily, 6 mice/group).

Administration route and administration frequency were the same as in Example 17.

General clinical observation, body weight, and tumor volume were conducted as in example 17.

Conclusion: 18G272 can significantly inhibit the tumor growth of mice bearing NCI-H596 (human lung adenosquamous carcinoma) at the dose of 30µg per mouse. Mice in 18G272 group had an upward trend in body weight and were well tolerated during treatment at the dose of 30µg per mouse. The results were shown in Figure 7 and Table 19.

**Table 19 Tumor growth and body weight of mice subcutaneously bearing NCI-H596**

| Groups | Tumor volume on D72 (mm³) | Body weight on D1 (g) | Body weight on D72 (g) |
|---|---|---|---|
| Model group | 560.84±70.19 | 19.22±0.54 | 23.95±0.74 |
| 18G272 group | 298.46±34.56* | 19.14±0.33 | 24.52±0.48 |

| | | | |
|---|---|---|---|
| Note: * : P < 0.05, vs Model group. | | | |

### Example 19 Efficacy of humanized bispecific antibody 19G294 in NOD/SCID mice subcutaneously bearing NCI-H226 human mesothelioma cell xenografts

Methods: 5×10⁶cells/0.1ml NCI-H226 cell suspension and 15×10⁷cells/0.1ml PBMC cell suspension were fully mixed in a volume ratio of 1:1 and inoculated subcutaneously into NOD/SCID mice aged 5 to 7 weeks weighing 18 to 22 g, 0.2mL per mouse.The animals were randomly divided into two groups according to body weight: Model group (PBS, 5 mice/group) and 19G294 group (60µg per mouse once daily, 5 mice/group). Administration route and administration frequency were the same as in Example 17.

General clinical observation, body weight, and tumor volume were conducted as in example 17.

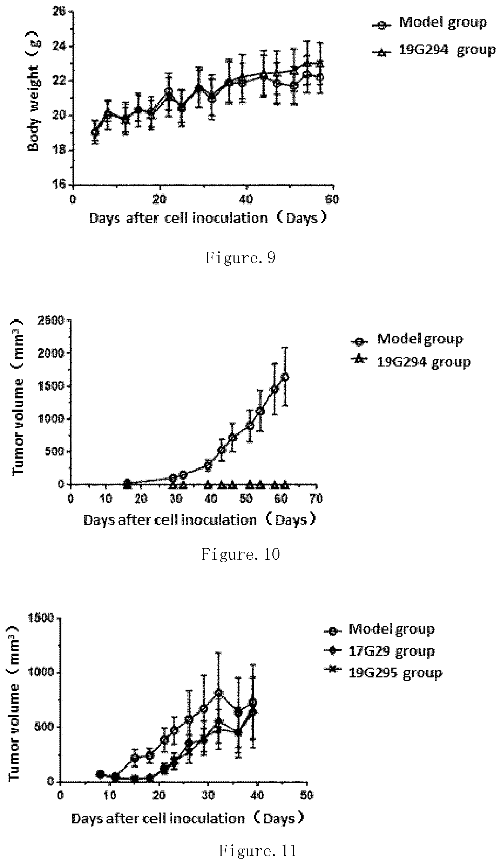

Conclusion: 19G294 can significantly inhibit the tumor growth of mice bearing NCI-H226 human lung adenosquamous carcinoma (mesothelioma) at the dose of 60µg per mouse, as shown in Figure 8. At the dose of 60µg per mouse , mice in 19G294 group had an upward trend in body weight and were well tolerated during treatment. The results were shown in Figure 9 and Table 20.

**Table 20 Tumor growth and body weight of mice subcutaneously bearing NCI-H226**

| Groups | Tumor volume on D57 (mm³) | Tumor weight on D57 (mg) | TGI a(%) | Body weight on D1 (g) | Body weight on D57 (g) |
|---|---|---|---|---|---|
| Model group | 653.13±74.92 | 372.58±56.92 | -- | 19.06±0.67 | 22.22±0.92 |
| 19G294 group | 228.60±74.30** | 127.18±47.60** | 66% | 19.28±0.60 | 23.00±1.19 |

| | | | | | |
|---|---|---|---|---|---|
| Note: * : P < 0.05, vs Model group. a: indicates TGI calculated according to tumor weight on day 57 after cell inoculation. | | | | | |

### Example 20 Efficacy of humanized bispecific antibody 19G294 in NOD/SCID mice subcutaneously bearing A498 human renal cancer cells xenografts

Methods: 1×10⁷cells/0.1ml A498 cell suspension and 1×10⁷ cells/0.1ml PBMC suspension were fully mixed in a volume ratio of 1:1 and inoculated subcutaneously into NOD/SCID mice aged 5 to 7 weeks weighing 18 to 22 g, 0.2mL per mouse. According to the body weight, the animals were randomly divided into two groups: Model group (PBS) and 19G294 group (30µg per mouse once daily), with 5 animals in each group.

Administration route and administration frequency were the same as in Example 17.

General clinical observation, body weight, tumor volume, and tumor weight were conducted as in example 17.

Conclusion: 19G294 can completely inhibit the tumor growth of mice bearing A498 human renal cell carcinoma at the dose of 30µg per mouse, as shown in Figure 10 and Table 21. Mice in 19G294 group did not show weight loss and were well tolerated during treatment at the dose of 30µg per mouse.

**Table 21 Tumor growth and body weight of mice subcutaneously bearing A498**

| Groups | Tumor volume on D61 (mm³) | Tumor weight on D61 (mg) | TGI ^{a} (%) | Body weight on D1 (g) | Body weight on D61 (g) |
|---|---|---|---|---|---|
| Model group | 1648.94±444.85 | 1105.04±342.68 | -- | 20.68±0.81 | 24.06±0.81 |
| 19G294 group | 0.00±0.00** | 0.00±0.00** | 100% | 20.50±0.50 | 24.18±0.93 |

| | | | | | |
|---|---|---|---|---|---|
| Note:* : P < 0.05, vs Model group; ** : P < 0.01, vs Model group. a: indicates TGI calculated according to tumor weight on day 61 after cell inoculation. | | | | | |

### Example 21 Efficacy of humanized bispecific antibodies 18G272 and 19G294 in NOD/SCID mice subcutaneously bearing OS-RC-2 human renal cancer cells xenografts

### Experiment 1:

Methods: 5×10⁶cells/0.1ml OS-RC-2 cell suspension and 1×10⁷ cells/0.1ml PBMC cell suspension were fully mixed in a volume ratio of 1:1 and inoculated subcutaneously into NOD/SCID mice aged 5 to 7 weeks weighing 18 to 22 g, 0.2mL per mouse. The animals were randomly divided into three groups according to the body weight: Model group (PBS), 17G29 group (15µg per mouse once daily), 19G295 group (15µg per mouse once daily). There were 8 animals in each group.

Administration route and administration frequency were the same as in example 17.

General clinical observation, body weight, and tumor volume were conducted as in example 17.

Conclusion: At the dose of 15µg per mouse , 17G29 and 19G295 can significantly inhibit tumor growth on D15, as shown in Figure 11. However, 17G29 and 19G295 could not inhibit tumor growth on D39, and there were no significant differences between them. In addition, mice subcutaneously bearing OS-RC-2 were prone to die during the test, while 17G29 and 19G295 both improved the median survival time (MST) of mice at the dose of 15µg per mouse, with no statistically significant difference.

**Table 22 Tumor growth and body weight of mice subcutaneously bearing OS-RC-2**

| Groups | Tumor volume on D15 (mm³) | Tumor volume on D39 (mm³) | Body weight on D1 (g) | Body weight on D15 (g) | Survival rate on D39 |
|---|---|---|---|---|---|
| Model group | 222.35±79.19 | 731.61±296.48 | 18.92±0.50 | 19.24±0.18 | 3/8 |
| 17G29 group | 30.48±15.81* | 633.78±248.84 | 18.91±0.51 | 19.74±0.40 | 3/8 |
| 19G295 group | 31.77±13.63* | 676.71±261.55 | 19.15±0.35 | 20.05±0.43 | 6/8 |

Note: * : P < 0.05, vs Model group; ** : P < 0.01, vs Model group; After D15, the animals in each group began to die or were euthanized due to tumor growth.

### Experiment 2:

Methods: 3×10⁶cells/0.1ml OS-RC-2 cell suspension and 6×10⁶ cells/0.1ml PBMC cell suspension were fully mixed in a volume ratio of 1:1 and inoculated subcutaneously into NOD/SCID mice aged 5 to 7 weeks weighing 18 to 22 g, 0.2mL per mouse. The animals were randomly divided into three groups according to the body weight: Model group (PBS, n=6), 18G272 group (30µg per mouse once daily, n=6), 19G295 group (30µg per mouse once daily, n=6).

Administration route and administration frequency were the same as in Example 17.

The general clinical observation, body weight, and tumor volume were conducted as in example 17.

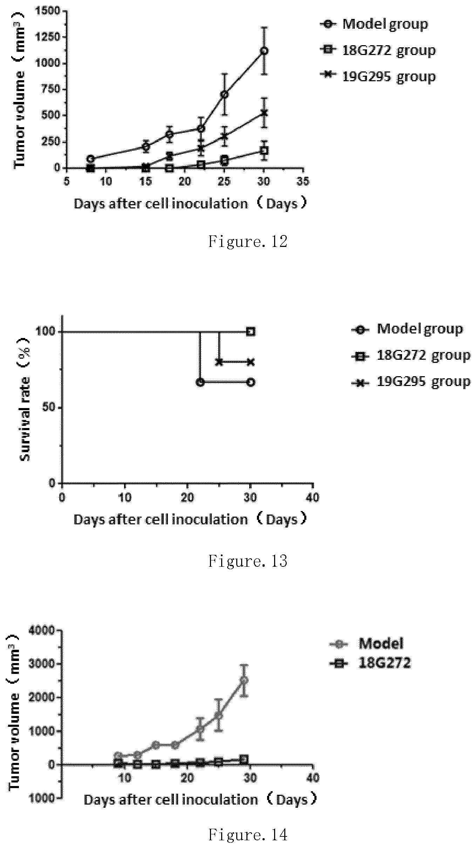

Conclusion: Compared with the model group, 18G272 can significantly inhibit the tumor growth of mice on D22 at the dose of 30µg per mouse. 19G295 had a trend of tumor inhibition on D22, but there was no significant difference compared with the model group. On D30, 18G272 still had a very efficacious inhibition of tumor growth in mice, with a very significant difference. 19G295 had a tendency to inhibit the tumor on D30, but there was no significant difference compared with the model group, as shown in Figure 12 and Table 23. Mice bearing subcutaneous OS-RC-2 were prone to die during the test, mice in 19G295 group died whereas none mice died in 18G272 group at the dose of 30µg per mouse, as shown in Figure 13 and Table 23. In conclusion, the anti-tumor effect of 18G272 was better than that of 19G295.

**Table 23 Tumor growth , and survival rate of mice subcutaneously bearing OS-RC-2**

| Groups | Tumor volume on D22 (mm³) | Tumor volume on D30 (mm³) | TGI ^{a} (%) | Body weight on D1 (g) | Body weight on D22 (g) | Survival rate on D31 |
|---|---|---|---|---|---|---|
| Model group | 376.36±107.37 | 1121.61±225.44 | | 17.37±0.92 | 12.93±1.53 | 2/3 |
| 18G272 group | 32.59±32.59** | 168.39±90.70** | 91.3% | 18.12±0.38 | 20.40±0.45 | 5/5 |
| 19G295 group | 190.86±69.80 | 528.05±140.97 | 49.3% | 18.06±0.34 | 18.22±1.11 | 4/5 |

| | | | | | | |
|---|---|---|---|---|---|---|
| Note: * : P < 0.05, vs Model group; ** : P < 0.01, vs Model group. After D22, the animals in each group began to die or were euthanized due to tumor growth, a: Indicates TGI calculated according to tumor volume on day 22 after cell inoculation. | | | | | | |

### Experiment 3:

Methods: 3×10⁶cells/0.1ml OS-RC-2 cell suspension and 6×10⁶ cells/0.1ml PBMC suspension were fully mixed in a volume ratio of 1:1 and inoculated subcutaneously into NOD/SCID mice aged 5 to 7 weeks weighing 18 to 22 g, 0.2mL per mouse. The animals were randomly divided into two groups according to the body weight: Model group (PBS) and 18G272 group (30µg per mouse once daily). There were six animals in each group.

Administration route and administration frequency were the same as in Example 17.

General clinical observation, body weight, tumor volume, and tumor weight detection were the same as in example 17.

Conclusion: 18G272 can significantly inhibit the tumor growth of mice bearing OS-RC-2 at the dose of 30µg permouse, as shown in FIG. 14 and Table 24. Mice bearing OS-RC-2 were prone to die during the test. Under the conditions of this test, 18G272 can significantly improve the median survival time (MST) of the mice at a dose of 30µg per mouse, reduce animal mortality, and mice in 18G272 group were well tolerated during the treatment. The results were shown in Table 24.

**Table 24 Tumor growth, body weight, and survival rate of mice subcutaneously bearing OS-RC-2**

| Groups | Tumor volume on D15 (mm3) | Tumor weight on D29 (mg) | TGI ^{a} (%) | Body weight on D1 (g) | Body weight on D15 (g) | Survival rate on D31 |
|---|---|---|---|---|---|---|
| Model group | 598.55±80.50 | 1656.15±147.85 | -- | 18.33±0.18 | 18.07±0.94 | 2/6 |
| 18G272 group | 12.87±12.87** | 96.80±38.41 | 94.2% | 18.30±0.19 | 19.43±0.67 | 6/6 |

| | | | | | | |
|---|---|---|---|---|---|---|
| Note:* : P < 0.05, vs Model group; ** : P < 0.01, vs Model group; After D15, the animals in each group began to die or were euthanized due to tumor growth, a: Indicates TGI was calculated according to tumor weight on day 29 after cell inoculation. | | | | | | |

### Experiment 4:

Methods: 3×10⁶cells/0.1ml OS-RC-2 cell suspension and 6×10⁶ human PBMC suspension were fully mixed in a volume ratio of 1:1 and inoculated subcutaneously into NOD/SCID mice aged 5 to 7 weeks weighing 18 to 22 g, 0.2mL per mouse. The animals were randomly divided into two groups according to the body weight: Model group (PBS) and 19G294 group (30µg per mouse once daily). There were 5 animals in each group.

Administration route and administration frequency were the same as in Example 17.

General clinical observation, body weight, and tumor volume were conducted as in example 17.

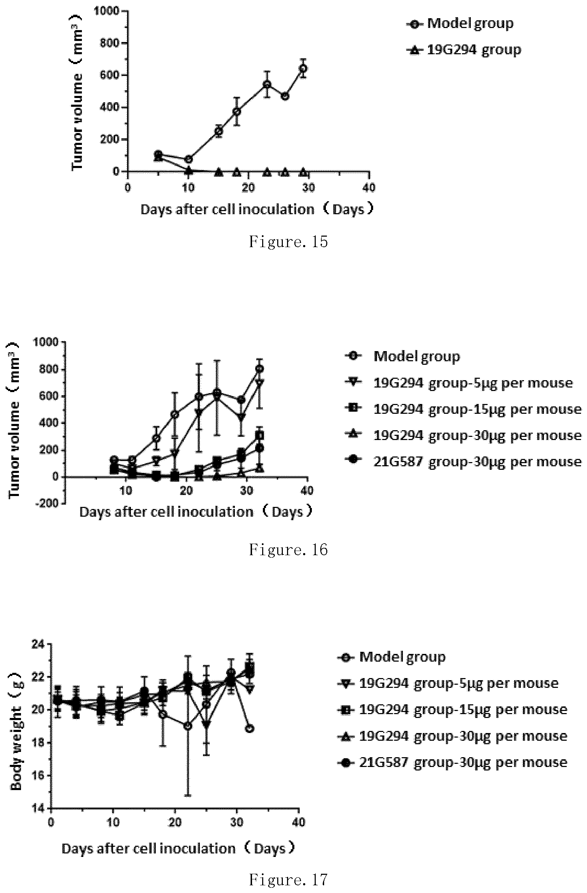

Conclusion: 19G294 can significantly inhibit the tumor growth of mice bearing OS-RC-2 at the dose of 30µg per mouse, as shown in Figure 15 and Table 25. Mice bearing OS-RC-2 were prone to die during the test. Under the conditions of this test, 19G294 can reduce the mortality of animals, prolong the survival time of animals, and mice in 19G294 group were well tolerated during the treatment. The results were shown in Table 25.

**Table 25 Tumor growth, body weight, and survival rate of mice subcutaneously bearing OS-RC-2**

| Groups | Tumor volume on D23 (mm³) | Tumor volume on D29 (mm³) | Body weight on D1 (g) | Body weight on D23 (g) | Survival rate on D29 |
|---|---|---|---|---|---|
| Model group | 545.91±80.93 | 645.40±56.68 | 19.04±0.85 | 16.22±1.49 | 2/5 |
| 19G294 group | 0.00±0.00** | 0.00±0.00** | 18.76±0.57 | 20.34±0.52* | 5/5 |

| | | | | | |
|---|---|---|---|---|---|
| Note:* : P < 0.05, vs Model group; ** : P < 0.01, vs Model group; Animals began to die or were euthanized after D23 | | | | | |

Combined with the results of above four experiments, the efficacy of 19G295 was equivalent to 17G29, while inferior to 18G272 in NOD/SCID mice subcutaneously bearing OS-RC-2 human renal cancer cell xenografts. 19G294 was similar to 18G272, and both could significantly inhibit or even completely inhibit tumor growth of mice bearing OS-RC-2. In terms of safety, 19G295 was similar to 17G29, inferior to 18G272, and 19G294 was similar to 18G272. In conclusion, 18G272 and 19G294 had better efficacy and safety than 17G29 in vivo.

### Example 22 Efficacy of different doses of humanized bipecific antibodies 19G294 and 21G587 in NOD/SCID mice subcutaneously bearing OS-RC-2 human renal cancer cell xenografts

Methods: 3×10⁶cells/0.1ml OS-RC-2 cell suspension and 6×10⁶ cells/0.1ml PBMC suspension were fully mixed in a volume ratio of 1:1 and inoculated subcutaneously into NOD/SCID mice aged 5 to 7 weeks weighing 18 to 22 g, 0.2mL per mouse. According to the body weight, the animals were randomly divided into five groups: Model group (PBS), 19G294 group (5µg per mouse once daliy), 19G294 group (15µg per mouse once daliy), 19G294 group (30µg per mouse once daliy), 21G587 group (30µg per mouse once daliy). There were three animals in each group.

Administration route and administration frequency were the same as in Example 17.

General clinical observation, body weight, tumor volume, and tumor weight detection were the same as in example 17.

Conclusion: 19G294 (15µg, 30µg per mouse) and 21G587 (30µg per mouse) could significantly inhibit the tumor growth of mice bearing OS-RC-2 . The inhibition of tumor growth by 19G294 was in dose-dependent. Mice bearing OS-RC-2 were prone to die or had significant weight loss during the test. Under the conditions of this test, 19G294(5, 15, 30µg/mouse) and 21G587(30µg/mouse) could improve the decline inhealth status of animals caused by tumor growth and reduce the mortality of animals, and animals in these groups were well tolerated. The results were shown in Figure 16, Figure 17, and Table 26.

**Table 26 Tumor growth, body weight, and survival rate of mice subcutaneously bearing OS-RC-2**

| Groups | Tumor volume on D18 (mm³) | Tumor weight on D33 (mg) | TGI ^{a} (%) | Body weight on D1 (g) | Body weight on D18 (g) | Survival rate on D33 |
|---|---|---|---|---|---|---|
| Model group | 467.29±160.94 | 471.60±0.00 | -- | 20.53±0.93 | 19.77±1.92 | 1/3 |
| 19G294 group - 5µg/mouse | 173.34±116.15 | 311.90±44.42 | 34 | 20.67±0.72 | 21.20±0.64 | 2/3 |
| 19G294 group - 15µg/mouse | 10.21±10.21 * | 138.87±38.01 | 71 | 20.67±0.67 | 20.77±0.48 | 3/3 |
| 19G294 group - 30µg/mouse | 0.00±0.00 * | 34.20±21.55 | 93 | 20.67±0.61 | 21.10±0.57 | 3/3 |
| 21G587 group - 30µg/mouse | 15.84±15.84 * | 122.40±61.17 | 74 | 20.60±0.51 | 21.03±0.43 | 3/3 |

| | | | | | | |
|---|---|---|---|---|---|---|
| Note:* : P < 0.05, vs Model group; ** : P < 0.01, vs Model group; Animals began to die or be euthanized after D18. a: Indicates TGI calculated according to tumor weight on day 33 after cell inoculation. | | | | | | |

### Example 23 Efficacy of 19G294(humanized bispecific antibody), axitinib combined with BAVENCIO, and YS110 in NOD/SCID mice subcutaneously bearing OS-RC-2 human renal cancer cell xenografts

Methods: 3×10⁶cells/0.1ml OS-RC-2 cell suspension and 6×10⁶ cells/0.1ml human PBMC suspension were fully mixed in a volume ratio of 1:1 and inoculated subcutaneously into NOD/SCID mice aged 5 to 7 weeks weighing 18 to 22 g, 0.2mL per mouse. According to the body weight, the animals were randomly divided into 5 groups: Model group (PBS), positive control-A (BAVENCIO, 1.8mg/mouse once; Axitinib, 30µg/mouse once), positive control-B (BAVENCIO, 1.8mg/mouse once; Axitinib, 60µg/mouse once), 19G294 (30µg/mouse), YS110 (205µg/mouse once); There were 3 to 4 animals in each group. Axitinib was administered by gavage. BAVENCIO, test molecules, and PBS were administered by intravenous injection. Axitinib, 19G294 and PBS were administered on the day of inoculation for 5 consecutive days. Two days after drug withdrawal, the second course of treatment was administered (5 consecutive days), once a day (10 doses total). BAVENCIO was administered once a week for 2 consecutive weeks starting on the day of inoculation (2 doses total). YS110 was administered twice a week for 2 weeks starting on the day of inoculation (4 doses total).

General clinical observation, body weight, tumor volume, and tumor weight detection were the same as those of example 17.

Conclusion: 19G294 significantly inhibited tumor growth of mice bearing OS-RC-2 at the dose of 30µg per mouse. BAVENCIO(1.8mg/mouse) combined with axitinib (30µg/mouse, 60µg/mouse) and YS110(205µg/mouse) did not significantly inhibit tumor growth of mice bearing OS-RC-2. Mice bearing OS-RC-2 were prone to die or had significant weight loss during the experimental period. Under the conditions of this experiment, 19G294(30µg/mouse) could improve the decline in health status of animals caused by tumor growth and reduce the mortality of animals, and mice in the group were well tolerated during the treatment. Neither BAVENCIO combined with axitinib (two dose groups) nor YS 110 had the above improvement effects. In conclusion, 19G294 had better efficacy than BAVENCIO combined with axitinib and YS 110 under the conditions of this experiment. The results were shown in Figure 18, Figure 19, and Table 27.

**Table 27 Tumor growth, body weight, and survival rate of mice subcutaneously bearing OS-RC-2**

| Groups | Tumor volume on D26 (mm3) | Tumor weight on D31 (mg) | TGI ^{a} (%) | Body weight on D1 (g) | Body weight on D26 (g) | Survival rate on D31 |
|---|---|---|---|---|---|---|
| Model group | 720.68±112.77 | 817.80±35.33 | -- | 21.63±0.84 | 16.27±1.28 | 3/3 |
| positive control-A | 528.14±35.33 | 656.50±0.00 | 20 | 21.50±0.54 | 17.45±1.01 | 1/4 |
| positive control-B | 390.03±108.54 | 435.30±175.60 | 47 | 21.60±0.40 | 19.05±1.82 | 3/4 |
| 19G294 group | 0.00±0.00 ** | 0.00±0.00** | 100 | 21.73±0.38 | 22.20±0.35 | 3/3 |
| YS110 group | 752.63±39.56 | -- | -- | 21.67±0.26 | 16.43±0.55 | 0/3 |

| | | | | | | |
|---|---|---|---|---|---|---|
| Note:* : P < 0.05, vs Model group; ** : P < 0.01, vs Model group; Animals began to die or be euthanized after D26. a: Indicated that TGI was calculated according to tumor weight on day 31 after cell inoculation. | | | | | | |

### Example 24 Efficacy of 19G294 (humanized bispecific antibody), axitinib combined with BAVENCIO , and YS110 in NOD/SCID mice subcutaneously bearing A498 human renal cancer cell xenografts

Methods: 7×10⁶ cells/0.1ml A498 cell suspension and 7×10⁶ cells/0.1ml PBMC suspension were fully mixed in a volume ratio of 1:1 and inoculated subcutaneously into NOD/SCID mice aged 5 to 7 weeks weighing 18 to 22 g, 0.2mL per mouse. According to the body weight, the animals were randomly divided into 5 groups: Model group (PBS), positive control-B (BAVENCIO, 1.8mg/mouse, Axitinib, 60µg/mouse), positive control-C (BAVENCIO, 1.8mg/mouse, Axitinib, 120/µg/mouse), 19G294 (30µg/mouse), YS110 (205µg/mouse), There were three animals in each group. Axitinib was administered by gavage. BAVENCIO, test molecules, and PBS were administered by intravenous injection. Axitinib, 19G294 and PBS were administered on the day of inoculation for 5 consecutive days. Two days after drug withdrawal, the second course of treatment was administered (5 consecutive days), once a day (10 doses total). BAVENCIO was administrated once every 2 weeks from the day of inoculation (once in total). YS110 was administered twice a week for 2 weeks starting on the day of inoculation (4 doses total).

General clinical observation, body weight, tumor volume, and tumor weight detection were the same as those of example 17.

Organ weight and organ coefficient: The animals were euthanized after the last examination. The organs of the mice were separated, rinsed with normal saline , blotted dry with filter paper, weighed and photographed. Organ coefficient = organ weight/body weight of mice × 100% (organ weight and body weight of mice were measured in g).

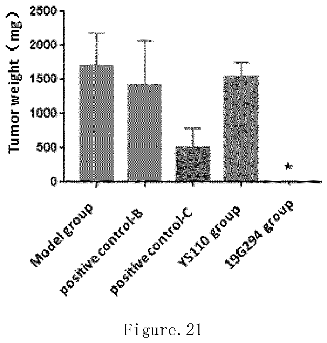

Conclusion: 19G294 could significantly inhibit the tumor growth of mice bearing A498 at the dose of 30µg per mouse. BAVENCIO(1.8mg/mouse) combined with axitinib (60µg/ mouse, 120µg/ mouse) and YS110(205µg/ mouse) did not significantly inhibit tumor growth of mice bearing A498. Mice in 19G294 (30µg/ drug), BAVENCIO combined with axitinib (two dose groups) and YS110 group were well tolerated during the treatment. 19G294(30µg/ mouse) and YS 110 had no significant effect on kidney, spleen, liver, lung and corresponding organ coefficient of mice. BAVENCIO combined with axitinib (two dose groups) had a significant effect on liver and/or liver coefficient, but had no significant effect on kidney, spleen, lung and corresponding organ coefficient of mice. In conclusion, under the conditions of this experiment, 19G294 had better efficacy than BAVENCIO combined with axitinib and YS 110, and had better safety than BAVENCIO combined with axitinib. The results were shown in Figs. 20, 21, and Tables 28 and 29.

**Table 28 Tumor growth, body weight, and survival rate of mice subcutaneously bearing A498**

| Groups | Tumor volume on D64 (mm³) | Tumor weight on D65 (mg) | TGI ^{a} (%) | Body weight on D1 (g) | Body weight on D64 (g) |
|---|---|---|---|---|---|
| Model group | 2,324.41±776.85 | 1,699.401483.70 | -- | 20.97±0.84 | 22.47±0.69 |
| positive control-B | 2,105.09±728.33 | 1,408.20±661.50 | 17 | 20.90±0.74 | 23.15±1.85 |
| positive control-C | 806.49±434.55 | 499.63±288.76 | 71 | 20.90±0.57 | 24.93±0.47 |
| YS110 group | 1,979.16±97.24 | 1,535.17±219.51 | 10 | 20.93±0.38 | 23.40±1.35 |
| 19G294 group | 0.00±0.00 * | 0.00±0.00 * | 100 | 20.93±0.22 | 22.77±0.41 |

| | | | | | |
|---|---|---|---|---|---|
| Note:* : P < 0.05, vs Model group; ** : P < 0.01, vs Model group. a: Indicates TGI was calculated according to tumor weight on day 65 after cell inoculation. | | | | | |

**Table 29-1 Organ weight and organ coefficient of mice subcutaneously bearing A498**

| Groups | Kidney weight (mg) | kidney coefficient (g/g) | Spleen weight (mg) | Spleen coefficient (g/g) |
|---|---|---|---|---|
| Model group | 240.43±26.28 | 1.070±1.02 | 54.87±5.21 | 0.244±0.15 |
| positive control-B | 259.35±24.55 | 1.119±0.17 | 54.35±8.85 | 0.233±0.20 |
| positive control-C | 282.40±13.79 | 1.131±0.37 | 63.50±5.13 | 0.254±0.17 |
| YS110 group | 254.70±13.30 | 1.089±0.07 | 60.10±8.91 | 0.255±0.27 |
| 19G294 group | 284.83±13.66 | 1.250±0.38 | 63.90±3.11 | 0.280±0.10 |

**Table 29-2 Organ weight and organ coefficient of mice subcutaneously bearing A498**

| groups | Liver weight (mg) | coefficient (g/g) | Lung weight (mg) | Lung coefficient (g/g) |
|---|---|---|---|---|
| Model group | 1,070.97±23.17 | 4.772±1.12 | 167.80±6.52 | 0.750±0.59 |
| positive control-B | 1,181.60±31.10* | 5.126±2.75 | 166.45±5.05 | 0.722±0.36 |
| positive control-C | 1,284.80±34.57** | 5.151±0.45* | 164.83±2.26 | 0.661±0.06 |
| YS110 group | 1,195.97±131.82 | 5.078±2.89 | 177.57±7.62 | 0.761±0.24 |
| 19G294 group | 1,177.80±52.22 | 5.169±1.51 | 149.23±5.88 | 0.655±0.16 |

| | | | | |
|---|---|---|---|---|
| Note: * : P < 0.05, vs Model group; **: P<0.01, vs Model group | | | | |

## Claims

1. An antibody or antigen-binding fragment specifically binding to human CD26, comprising HCDR1 shown as SEQ ID NO: 1, HCDR2 shown as SEQ ID NO:2, and HCDR3 shown as SEQ ID NO:3; as well as LCDR1 shown as SEQ ID NO:4, LCDR2 consisting of Arg Met Ser, and LCDR3 shown as SEQ ID NO:5.

2. The antibody or antigen-binding fragment of claim 1, comprising a variable region of heavy chain that is at least 90%, 91%, 92%, 93%, 94%, 95%, 96%, 97%, 98%, or 99% identical to the amino acid sequence of SEQ ID NO:6 or SEQ ID NO:8,and a variable region of light chain that is at least 90%, 91%, 92%, 93%, 94%, 95%, 96%, 97%, 98%, or 99% identical to the amino acid sequence of SEQ ID NO:7 or SEQ ID NO:9.

3. The antibody or antigen-binding fragment of claim 2, wherein one, two, three, four, five, six, seven, eight, nine or ten amino acids of the sequence shown as SEQ ID NO:6, SEQ ID NO:7, SEQ ID NO:8 or SEQ ID NO:9 are inserted, deleted or substituted, the amino acid substitutions are conservative amino acid substitutions.

4. The antibody or antigen binding fragment of claim 2, comprising a variable region of heavy chain shown as SEQ ID NO:6 and a variable region of light chain shown as SEQ ID NO:7;Or comprising a variable region of heavy chain shown as SEQ ID NO:8 and a variable region of light chain shown as SEQ ID NO:9.

5. A bispecific T cell engager comprising the sequence of an antibody or antigen-binding fragment targeting CD26, which is formed by connecting two antigen-specific single-chain variable fragments (scFv) through a linker; One scFv targeting CD26, is formed by connecting a variable region of heavy chain targeting CD26 and a variable region of light chain targeting CD26 through a linker; Another scFv targeting CD3, is formed by connecting a variable region of heavy chain targeting CD3 and a variable region of light chain targeting CD3 through a linker ;
The scFv targeting CD26 comprises HCDR1 shown as SEQ ID NO: 1, HCDR2 shown as SEQ ID NO:2, and HCDR3 shown as SEQ ID NO:3; As well as LCDR1 shown as SEQ ID NO:4, LCDR2 consisting of Arg Met Ser, and LCDR3 shown as SEQ ID NO:5.

6. The bispecific T cell engager (BITE) comprising the sequence of an antibody or antigen-binding fragment targeting CD26 of claim 5, the scFv targeting CD26 comprises a variable region of heavy chain that is at least 90%, 91%, 92%, 93%, 94%, 95%, 96%, 97%, 98%, or 99% identical to the amino acid sequence of SEQ ID NO:6 or SEQ ID NO:8, and a variable region of light chain that is at least 90%, 91%, 92%, 93%, 94%, 95%, 96%, 97%, 98%, or 99% identical to the amino acid sequence of SEQ ID NO:7 or SEQ ID NO:9.

7. The bispecific T cell engager (BITE) comprising the sequence of an antibody or antigen-binding fragment targeting CD26 of claim 6, the scFv targeting CD26 comprises a sequence wherein one, two, three, four, five, six, seven, eight, nine, or ten amino acids of the sequence shown as SEQ ID NO:6, SEQ ID NO:7, SEQ ID NO:8, or SEQ ID NO:9 are inserted, deleted, or substituted, and such amino acid substitutions are conservative amino acid substitutions.

8. The bispecific T cell engager (BITE) comprising the sequence of an antibody or antigen-binding fragment targeting CD26 of claim 6, the scFv targeting CD26 comprises a variable region of heavy chain shown as SEQ ID NO:6 and a variable region of light chain shown as SEQ ID NO:7; Or comprises a variable region of heavy chain shown as SEQ ID NO:8 and a variable region of light chain shown as SEQ ID NO:9.

9. The bispecific T cell engager (BITE) comprising the sequence of an antibody or antigen-binding fragment targeting CD26 of claim 5, the amino acid sequence of the scFv targeting CD3 is derived from OKT-3, L2K, TR66, UCHT1, SP34, IORT3, Catumaxomab, Blinatumomab,or Solitomab.

10. The bispecific T cell engager comprising the sequence of an antibody or antigen-binding fragment targeting CD26 of claim 5, the linker connecting the variable region of heavy chain and the variable region of light chain in scFv is selected from linkers commonly used in the art, KESGSVSSEQLAQFRSLD, EGKSSGSGSESKST, GSTSGGGSGGGGSS, GSTSGSGKPGSGEGSTKG or (GGGGS)n, where n is an integer from 1 to 5.

11. The bispecific T cell engager comprising the sequence of an antibody or antigen-binding fragment targeting CD26 of claim 10, the sequence of the linker connecting the variable region of heavy chain and variable region of light chain in scFv is shown as SEQ ID NO: 12.

12. The bispecific T cell engager comprising the sequence of an antibody or antigen-binding fragment targeting CD26 of claim 5, the linker connecting two scFvs is selected from commonly used linkers in the art, such as (GGGGS)n, where n is an integer from 1 to 5.

13. The bispecific T cell engager comprising the sequence of an antibody or antigen-binding fragment targeting CD26 of claim 5, the linker connecting two scFvs is selected from commonly used linkers in the art, shown as SEQ ID NO: 13.

14. The bispecific T cell engager comprising the sequence of an antibody or antigen-binding fragment targeting CD26 of claim 5, the amino acid sequence of which is shown as SEQ ID NO: 14 or 15.

15. A nucleotide acid sequence encoding the amino acid sequence of the antibody or antigen-binding fragment of any one of claims 1-14.

16. A vector comprising the nucleotide sequence of claim 15.

17. Host cell comprising the vector of claim 15.

18. A pharmaceutical composition comprising the antibody or antigen-binding fragment of any one of claims 1-14.

19. A method for treating a CD26-highly expressing tumor, comprising administering to the patient an effective amount of any one of the antibody or antigen-binding fragments of claims 1-14;Such tumors include, but are not limited to kidney cancer, mesothelioma, lung cancer, liver cancer, prostate cancer.
